(19) Europäisches Patentamt
European Patent Office
Office européen des brevets

(11) EP 0 572 489 B1

(12) EUROPÄISCHE PATENTSCHRIFT

(45) Veröffentlichungstag und Bekanntmachung des Hinweises auf die Patenterteilung:
**04.09.1996 Patentblatt 1996/36**

(21) Anmeldenummer: **92905423.7**

(22) Anmeldetag: **19.02.1992**

(51) Int. Cl.⁶: **C07J 71/00**, C07J 9/00, C07J 51/00 // C07C401/00

(86) Internationale Anmeldenummer:
**PCT/DE92/00128**

(87) Internationale Veröffentlichungsnummer:
**WO 92/14746 (03.09.1992 Gazette 1992/23)**

# (54) AUSGANGSVERBINDUNGEN ZUR HERSTELLUNG VON CALCITRIOL SOWIE DESSEN ABKÖMMLINGEN, VERFAHREN ZUR HERSTELLUNG DIESER AUSGANGSVERBINDUNGEN SOWIE ZWISCHENPRODUKTE FÜR DIESES VERFAHREN

STARTING COMPOUNDS FOR PREPARING CALCITRIOL AND ITS DERIVATIVES, METHOD FOR PREPARING THESE STARTING COMPOUNDS AND INTERMEDIATE PRODUCTS FOR THIS METHOD

COMPOSES DE DEPART POUR LA FABRICATION DE CALCITRIOL ET DE SES DERIVES, PROCEDE D'OBTENTION DE CES COMPOSES DE DEPART ET PRODUITS INTERMEDIAIRES POUR CE PROCEDE

(84) Benannte Vertragsstaaten:
**AT BE CH DE DK ES FR GB GR IT LI LU NL SE**

(30) Priorität: **19.02.1991 DE 4105503**

(43) Veröffentlichungstag der Anmeldung:
**08.12.1993 Patentblatt 1993/49**

(73) Patentinhaber: **JENAPHARM GmbH**
**D-07745 Jena (DE)**

(72) Erfinder:
- **SCHÖNECKER, Bruno**
  **D-6900 Jena (DE)**
- **DROESCHER, Peter**
  **D-5300 Weimar (DE)**
- **ADAM, Günter**
  **D-4020 Halle (DE)**
- **MARQUARDT, Volker**
  **D-4020 Halle (DE)**
- **WALTHER, Dirk**
  **D-6902 Jena-Lobeda (DE)**
- **FISCHER, Reinald**
  **D-6900 Jena (DE)**
- **PROUSA, Richard**
  **D-6902 Jena-Lobeda (DE)**

(56) Entgegenhaltungen:

| | |
|---|---|
| **EP-A- 0 321 572** | **DD-A- 286 361** |
| **DD-A- 288 608** | **DE-A- 2 546 715** |
| **GB-A- 1 496 319** | **US-A- 4 022 768** |

- **DDR-288608**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.13.4/3.4

## Beschreibung

Die vorliegende Erfindung betrifft neue Ausgangsverbindungen zur Herstellung von Calcitriol (1α,25-Dihydroxycholecalciferol),

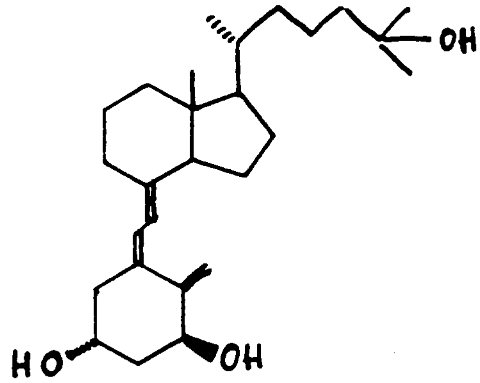

sowie dessen Abkömmlingen, die u.a. in der C-17-Seitenkette verglichen mit Calcitriol Modifikationen aufweisen.

Calcitriol besitzt ausgeprägte Wirkung auf den Calcium- und Phosphatstoffwechsel und daneben proliferationshemmende und zelldifferenzierende Wirkung (H. F. De Luca, The Metabolism and Function of Vitamin D in Biochemistry of Steroid Hormones, Hrsg. H.L.J. Makin, 2nd Edition, Blackwell Scientitic Publications 1984, S. 71-116). Bei verschiedenen seitenketten-modifizierten Calcitriolderivaten wurde beobachtet, daß diese überraschenderweise ein günstigeres Wirkungsspektrum als Calcitrol aufweisen; deren Effekte auf den Calcium- und Phosphatstoffwechsel sind nämlich gegenüber Calcitriol deutlich abgeschwächt, während die proliferationshemmenden und zelldifferenzierenden Wirkungen annähernd erhalten bleiben.

Beispielhaft seien hier die in der EP-A-0387077 beschriebenen 19-Nor-Vitamin D-Verbindungen und die in der internationalen Patentanmeldung WO 87/-00834 enthaltenen 24-Hydroxy-Vitamin-D-Analoga genannt.

Die beiden deutschen Patentanmeldungen P 39 33 034.6 und P 40 34 730.3 haben ebenfalls seitenketten-modifizierte Vitamin D-Derivate mit dissoziiertem Wirkungsspektrum zum Inhalt; sie lassen sich unter folgender Formel zusammenfassen:

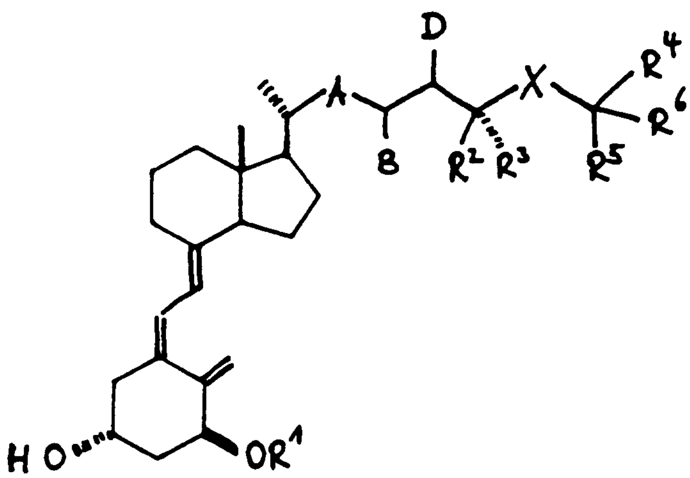

worin

| | |
|---|---|
| $R^1$ | ein Wasserstoffatom oder eine Acylgruppe mit 1 bis 9 Kohlenstoffatomen, |
| $R^2$ | oder $R^3$ eine Hydroxy- oder Acyloxygruppe mit 1 bis 9 Kohlenstoffatomen, und der jeweils andere Substituent ein Wasserstoffatom oder $R^2$ und $R^3$ gemeinsam ein Sauerstoffatom, |
| $R^4$ und $R^5$ | unabhängig voneinander jeweils einen linearen oder verzweigten Alkylrest mit bis zu 4 Kohlenstoffatomen, eine Trifluormethylgruppe oder gemeinsam einen mit dem tertiären Kohlenstoffatom |

gebildeten gesättigten, ungesättigten oder aromatischen carbocyclischen oder unter Einschluß von 1 oder 2 N-, O- oder S-Atomen heterocyclischen 3-, 4-, 5- oder 6-gliedrigen Ring,

$R^6$ ein Wasserstoffatom,

B und D entweder jeweils ein Wasserstoffatom oder gemeinsam eine zweite Bindung (E-konfigurierte Doppelbindung) und entweder

A eine direkte Bindung zwischen den Kohlenstoffatomen 20 und 22 oder eine Methylenbrücke ($-CH_2-$) zwischen den Kohlenstoffatomen 20 und 22 und

X einen Alkylenrest $-(CH_2)_n-$ oder einen Alkylenoxyrest $-(CH_2)_nO-$ mit n = = 1 bis 3

bedeuten.

Durch die Dissoziation der Eigenschaften werden Hypercalcämieerscheinungen durch Überdosierung bzw. Nebenwirkungen bei erforderlicher hoher Dosierung verringert.

Die in der P 39 33 034.6 und P 40 34 730.3 beschriebenen Vitamin D-Derivate werden durch Umsetzung des C-22- bzw. C-23- Aldehyds mit dem entsprechenden Wittig-Horner-Reagens sowie gegebenenfalls nachfolgender Hydrierung der Doppelbindung, so daß dann B und D je ein Wasserstoffatom bedeuten, erhalten.

Aufgabe der vorliegenden Erfindung ist es, neue Ausgangsverbindungen zur Herstellung von Calcitriol sowie von seitenketten-modifizierten Abkömmlingen von Calcitriol zur Verfügung zu stellen sowie ein Verfahren zur Herstellung dieser und weiterer, bereits bekannter Ausgangsverbindungen.

Die neuen Ausgangsverbindungen sollen dabei einfach und kostengünstig in guten Ausbeuten zugänglich sein.

Diese Aufgabe wird durch die Bereitstellung der neuen Ausgangsverbindungen der allgemeinen Formel Ia,

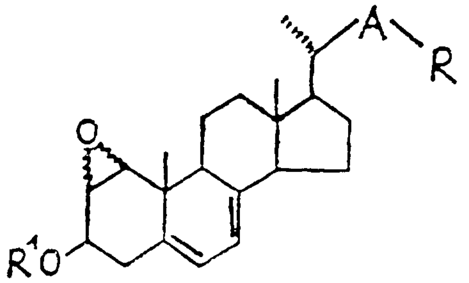

(Ia),

worin

die 1,2-Epoxygruppe α- oder β-ständig ist,

und

$R^1$ ein Wasserstoffatom, eine gerad- oder verzweigtkettige Alkyl-, eine alkylsubstituierte Silyl-, eine Acyl-, Aroyl-, Alkoxycarbonyl- oder Alkoxyalkylgruppe jeweils mit 1 bis 6 Kohlenstoffatomen im Alkyl-, Acyl-bzw. Alkoxyrest,

A eine Methylengruppe und

R den Rest $-(CH_2)_n-CH_2-CR^2R^3OH$, in welchem n für eine ganze Zahl von 1 bis 7, $R^2$ und $R^3$ unabhängig voneinander jeweils für ein Wasserstoffatom, eine gerad- oder verzweigtkettige Alkylgruppe mit 1 bis 4 Kohlenstoffatomen oder $R^2$ und $R^3$ gemeinsam mit dem endständigen Kohlenstoffatom der Seitenkette für einen Cyclopropyl-, -butyl-, -pentyl-oder -hexylring stehen,

bedeuten, sowie durch ein neues Verfahren zur Herstellung dieser Verbindungen und von 3β-Alkyloxycarbonyloxy-1α,2α-epoxy-5,7-pregnadienen der allgemeinen Formel Ib, die abweichend von den Verbindungen der allgemeinen Formel Ia als Gruppe A eine direkte Bindung und als Substituenten R einen Carbaldehydrest aufweisen, gelöst.

Solche Verbindungen der allgemeinen Formel Ib sind bereits aus der EP-A 0 321 572 bekannt.

Die neuen Ausgangsverbindungen lassen sich nach bekannten Verfahren der Vitamin D-Synthese zu wertvollen Endprodukten mit dem eingangs erwähnten dissoziierten Wirkungsprofil weiterverarbeiten. Steht R für einen Carbaldehydrest wird durch Umsetzung mit einem entsprechenden Wittig-Reagens die gewünschte Seitenkette eingeführt, die (noch) eine Doppelbindung enthält (Calverley et al.)

Die Doppelbindung kann katalytisch hydriert werden. Durch Öffnung des Epoxids, beispielweise mit Lithiumaluminiumhydrid und anschließende Bestrahlung sowie Abspaltung vorhandener Schutzgruppen und Veretherung oder Veresterung freier Hydroxygruppen gelangt man zu den letztendlich gewünschten Vitamin D-Strukturen.

Ausgangsprodukt zur Herstellung der erfindungsgemäßen Ausgangsverbindungen der allgemeinen Formel Ia sowie zur neuartigen Herstellung der Verbindungen Ib, die abweichend von den Verbindungen der allgemeinen Formel Ia als Gruppe A eine direkte Bindung und als Substituenten R einen Carbaldehydrest aufweisen (Ia und Ib zusammengenommen als Verbindungen der allgemeinen Formel I bezeichnet), ist das bekannte (20S)-20-Hydroxymethyl-1,4-pre-

gnadien-3-on, (II)

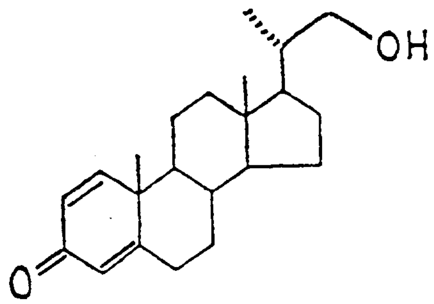

(II),

welches gemäß vorliegender Erfindung mit einem Alkalisalz eines aliphatischen $C_1$- bis $C_5$-Alkohols in etherischer Lösung zum (20S)-20-Hydroxymethyl-1,5-pregnadien-3-on (Formel III)

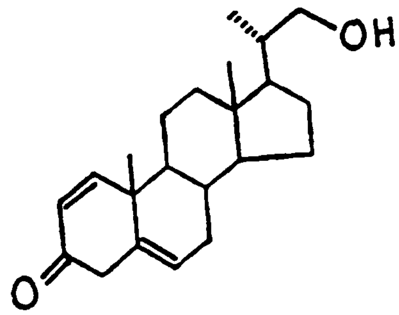

(III),

isomerisiert,

und dann entweder a), wenn in der herzustellenden Verbindung der allgemeinen Formel I A eine Methylengruppe und R den Rest $-(CH_2)_n-CH_2-CR^2R^3OH$ bedeuten soll,
diese Verbindung der Formel III mit einem aliphatischen oder aromatischen Sulfonsäurechlorid der allgemeinen Formel $R^4-SO_2-Cl$ ($R^4$ = Phenyl-oder $C_1$-bis $C_4$-Alkylrest) unter Zusatz einer Base unterhalb oder bei Raumtemperatur in die entsprechende 20-Sulfonyloxymethyl-Verbindung der allgemeinen Formel IVa

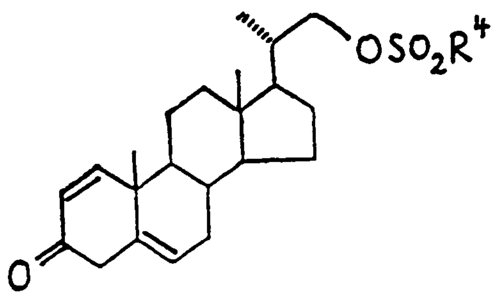

(IVa)

überführt,
und diese Verbindung mit einem komplexen Metallhydrid in alkoholischer und/oder etherischer Lösung zur entsprechenden 3β-Hydroxyverbindung der Formel Va

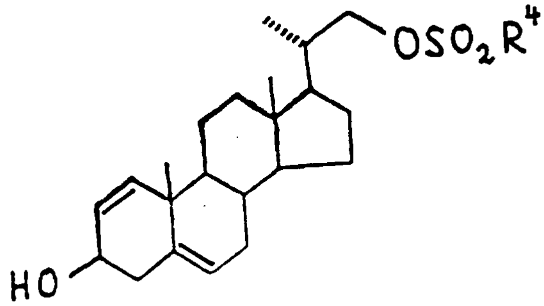

(Va)

reduziert, dann in dieser Verbindung, gegebenenfalls nach Überführung des Tosylats in das entsprechende Bromid oder Jodid, die Seitenkette entweder durch bekannte Verfahren oder durch Umsetzung mit einem Nickelchelatkomplex der allgemeinen Formel VI

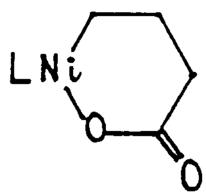

(VI)

worin

L einen 2,2'-Dipyridylrest (dipy) oder dessen Alkylsubstitutionsprodukte oder Ethylendiamin bzw. dessen N-alkylierte Derivate bedeutet, in organischen Lösungsmitteln oder Lösungsmittelgemischen bei einer Reaktionstemperatur zwischen Raumtemperatur und 55°C und unter Zusatz eines wasserfreien Mangan(II)-salzes, wobei für L=dipy die Reaktion unter heterogenen Bedingungen und für L=Ethylendiamin bzw. dessen N-alkylierte Derivate in homogener Phase bei gleichzeitiger Einwirkung von Ultraschall durchgeführt wird und die Reaktionsprodukte sauer hydrolysiert werden, unter Erhalt einer Verbindung der allgemeinen Formel VIIa

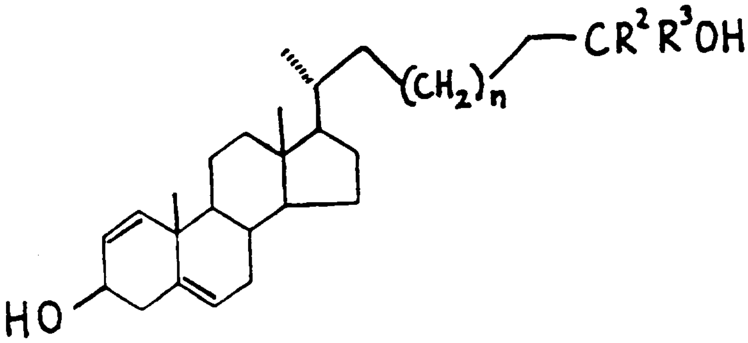

(VIIa)

worin $R^2$ und $R^3$ die bereits angegebene Bedeutung haben, bzw. im Falle, daß mit VI umgesetzt wurde, eine Verbindung der allgemeinen Formel VIIa'

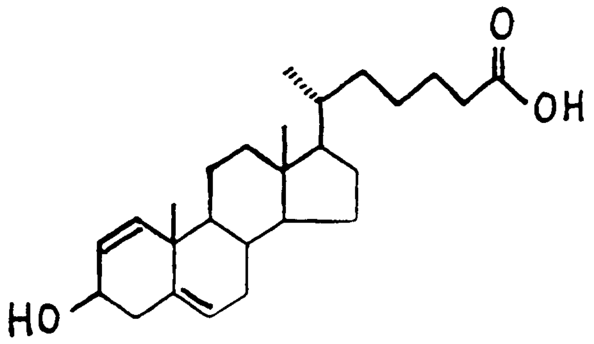

(VIIa')

deren Säuregruppe gegebenenfalls derivatisiert werden kann, aufgebaut, und anschließend eine Verbindung der allgemeinen Formel VIIa oder VIIa', im Fall der Verbindung der allgemeinen Formel VIIa' nach deren doppelten Grignardierung mit einer Verbindung $R^{23}MgHal$ ($R^{23}=R^2=R^3$; Hal=Cl, Br, J), zu einer Verbindung der allgemeinen Formel VIIIa

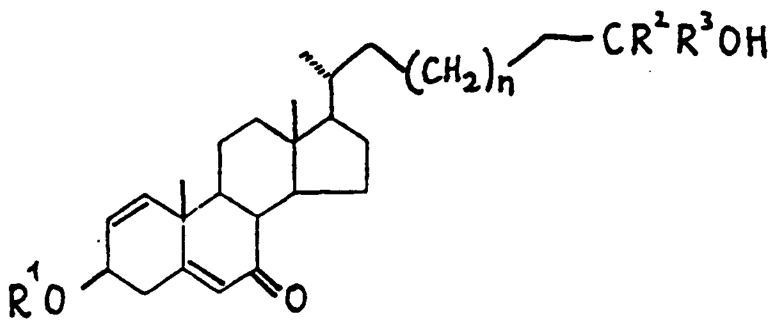

(VIIIa)

nach Schutz der sekundären OH-Gruppe, oxidiert und diese gegebenenfalls, wenn nicht $R^1$ bereits eine alkylsubstituierte Silylgruppe bedeutet, über die freie 3-Hydroxyverbindung in den entsprechenden 3-Silylether überführt und dieser zu einer Verbindung der allgemeinen Formel IXa

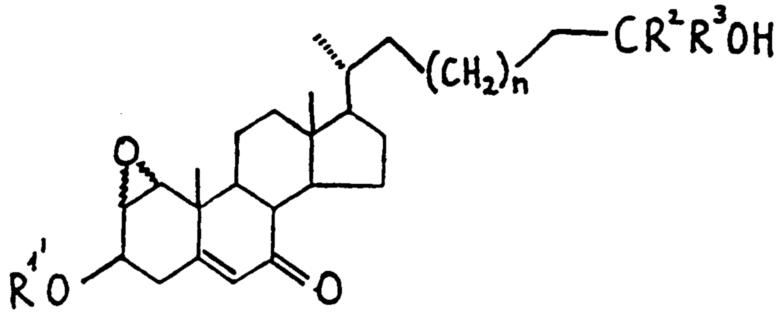

(IXa)

worin $R^{1'}$ den alkylsubstituierten Silylrest bedeutet (1,2α-Epoxid) oder, wenn nicht $R^1$ bereits eine Acyl- oder Aroylgruppe bedeutet, über die freie 3-Hydroxyverbindung in die entsprechende 3-Acyloxyverbindung überführt, und diese zu einer Verbindung der allgemeinen Formel IXa, worin $R^{1'}$ eine Acyl- oder Aroylgruppe bedeutet (1,2β-Epoxid) epoxidiert, oder die Verbindung der allgemeinen Formel VIIIa, worin $R^1$ eine Acyl- oder Aroylgruppe bedeutet, mit einer positiviertes Halogen, insbesondere positiviertes Brom enthaltenden Verbindung in eine Verbindung der allgemeinen Formel XI

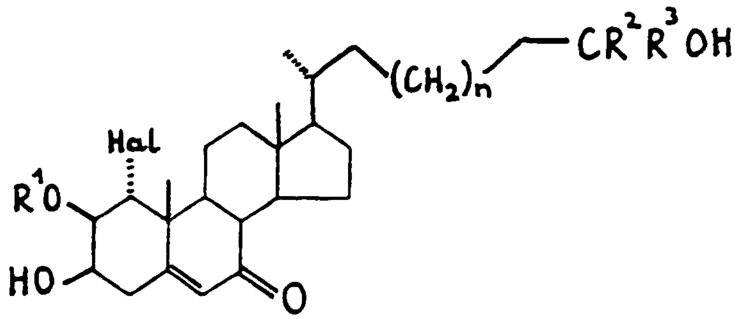

(XI)

überführt und diese mit einer schwachen Base in ein 1,2β-Epoxid der allgemeinen Formel IXaβ

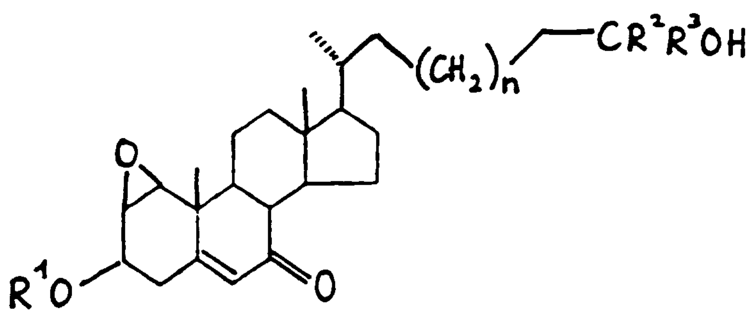

(IXaβ)

umgewandelt, gegebenenfalls in IXa der 3-Silylether gespalten und über die freie 3-Hydroxyverbindung gegebenenfalls in die entsprechende 3-Acyloxyverbindung (IXa mit $R^{1'}$ = Acyl) umgewandelt und mit Tosylhydrazin $CH_3$-$C_6H_4$-$SO_2$-NH-$NH_2$ in das Tosylhydrazid der allgemeinen Formel Xa

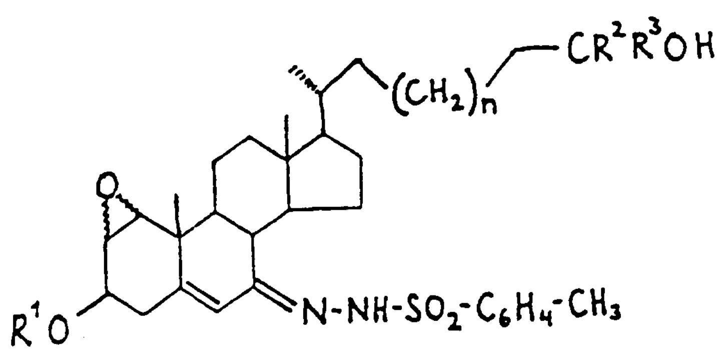

(Xa)

überführt und dieses mit Lithiumhydrid zu einer Verbindung der allgemeinen Formel Ia

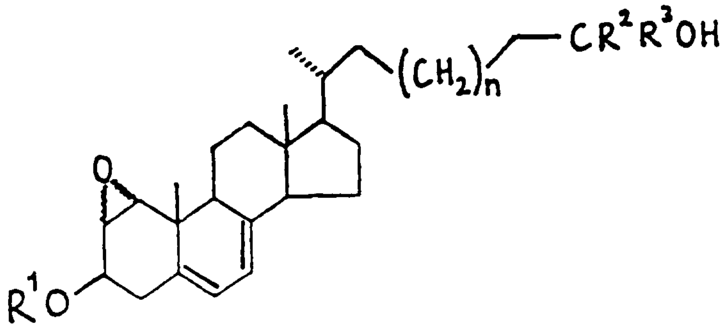

(Ia)

umgesetzt

oder b), wenn in der herzustellenden Verbindung der allgemeinen Formel I A eine direkte Bindung und R einen Carbaldehydrest -CHO bedeuten soll, in der Verbindung der Formel III eine leicht abspaltbare Schutzgruppe $R^x$ unter Erhalt des entsprechenden (20S)-20-substituierten-Methyl-1,5-pregnadien-3-on IVb

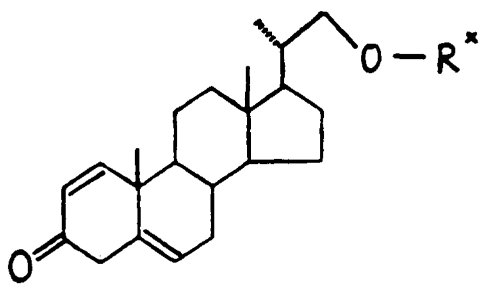

(IVb)

eingeführt,

und dieses mit einem komplexen Metallhydrid analog dem Reaktionsschritt IVa → Va zur entsprechenden 3-Hydroxyverbindung der allgemeinen Formel Vb

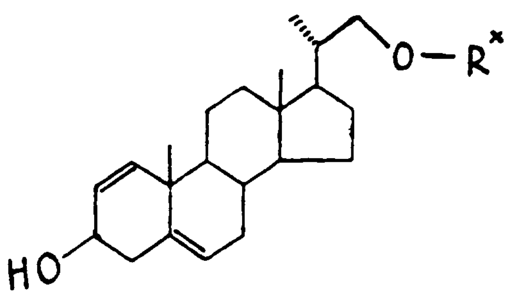

(Vb)

reduziert und darin die 3-Hydroxygruppe mit einem Alkoxycarbonylchlorid der allgemeinen Formel R"OC(O)Cl (R" ist ein gerad-oder verzweigtkettiger Alkylrest mit 1 bis 6 Kohlenstoffatomen) in Gegenwart einer Base verestert und anschließend der erhaltene Ester analog dem Reaktionsschritt VIIa bzw. VIIa'→VIIIa in Allylposition zur entsprechenden Verbindung der allgemeinen Formel VIIIb

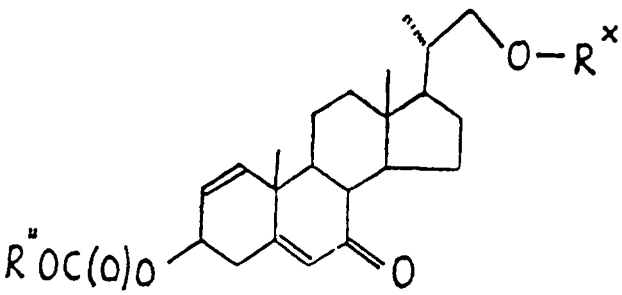

(VIIIb)

oxidiert,

diese Verbindung analog dem Reaktionsschritt VIIIa→IXa zur entsprechenden Verbindung der allgemeinen Formel IXb

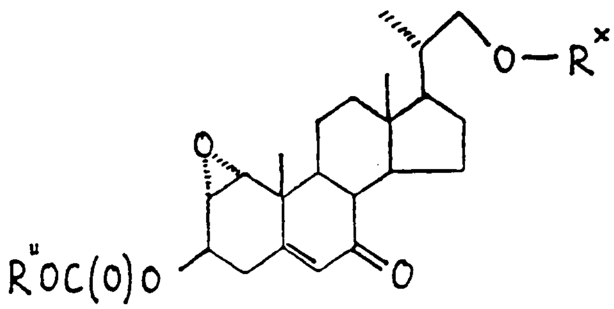

(IXb)

epoxidiert,

diese Verbindung analog dem Reaktionsschritt IXa→Xa mit Tosylhydrazon in das entsprechende Tosylhydrazid der allgemeinen Formel Xb

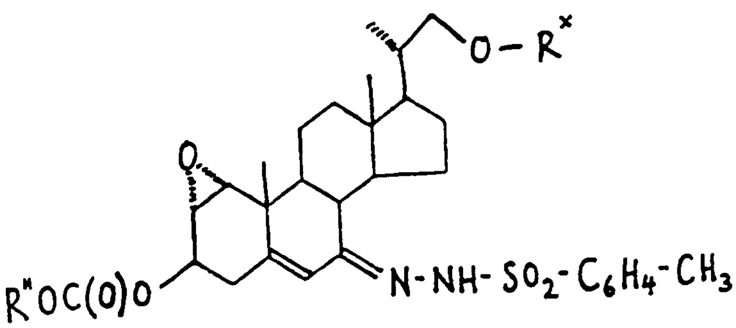

(Xb)

überführt, dieses anschließend analog dem Reaktionsschritt Xa→Ia mit Lithiumhydrid zu einer Verbindung der allgemeinen Formel Ib'

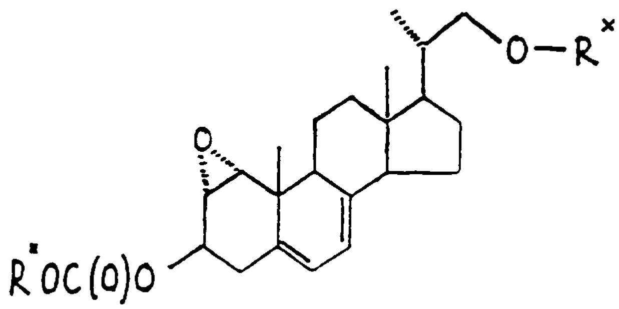

(Ib')

umgesetzt,
die Schutzgruppe $R^x$ unter milden Bedingungen abgespalten, die gebildete 20-Hydroxymethylverbindung der allgemeinen Formel Ib" schließlich zum entsprechenden 20-Aldehyd der allgemeinen Formel Ib

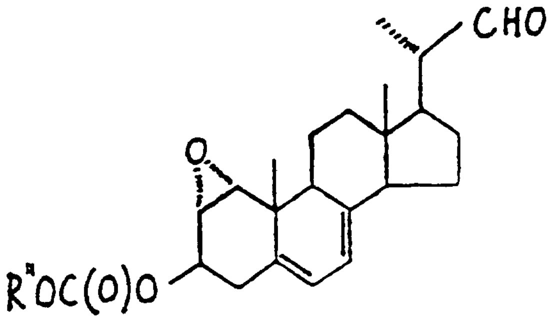

(Ib)

oxidiert und anschließend gewünschtenfalls der 3-Alkoxycarbonylester durch gängige Verfahren in einen anderen, vorstehend unter $R^1$ genannten Substituenten umgewandelt wird.

Die Veresterung von 3β,25-Dihydroxy-cholesta-1,5-dien-7-on mit Adamantoylchlorid unter Erhalt von 3β-Adamantoyloxy-25-hydroxy-cholesta-1,5-dien-7-on ist bereits in DD 288 608 A5 beschrieben.

Das erfindungsgemäße Verfahren ist in dem nachstehenden Schema zusammengefaßt.

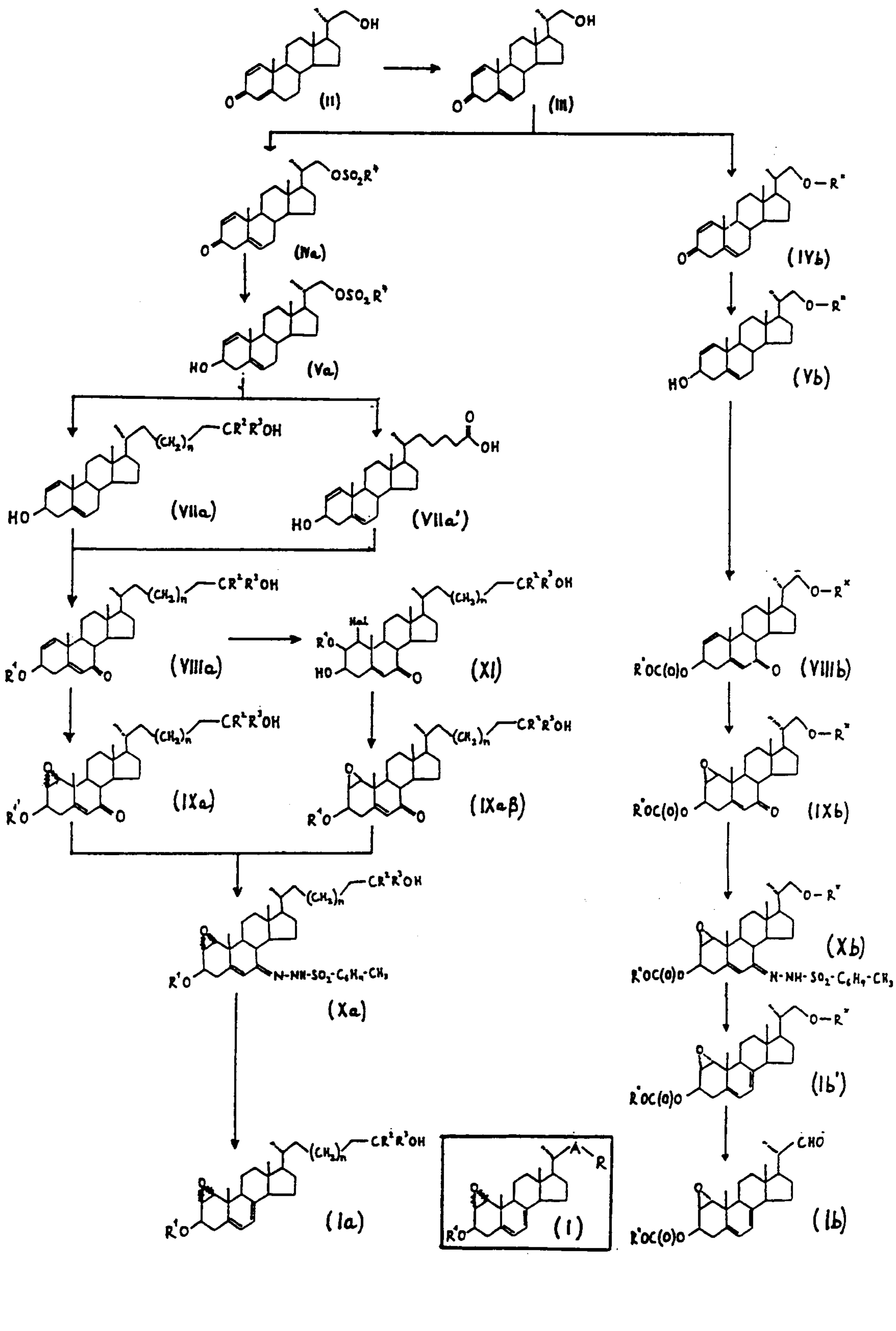

Die Isomerisierung der Verbindung der allgemeinen Formel II, (20S)-20-Hydroxymethyl-1,4-pregnadien-3-on, die durch mikrobiellen Abbau von Sterolen (DD-WP 139 859, DD-WP 140 478) gut zugänglich ist, zur Verbindung der allgemeinen Formel III ist bereits bekannt (DE-OS 27 46 107). Für die Isomerisierung werden relativ teure dipolar-aprotische Lösungsmittel (Dimethylsulfoxid, Hexamethylphosphorsäuretriamid) und Kalium-tert.-butanolat bzw. Natriumhydrid (Helv. Chim. Acta 64 (1981) 1870) eingesetzt.

Es wurde nunmehr gefunden, daß sich das 3-Keto-1,4-dien-System in II viel günstiger mit Alkalisalzen aliphatischer $C_1$- bis $C_5$-Alkohole in Ethern, vorzugsweise wird Kaliumbutan-2-olat in Tetrahydrofuran (DD-WP 114807) verwendet, zum 3-Keto1,5-dien-System der Verbindung III isomerisieren last. Je nach letztendlich gewünschtem Produkt der Formel I wird die Verbindung III weiterverarbeitet.

Um zu Produkten der allgemeinen Formel Ia zu gelangen, wird III mit aliphatischen oder aromatischen Sulfonsäurechloriden unter Zusatz einer Base unterhalb oder bei Raumtemperatur in die entsprechenden 20-Sulfonyloxymethyl-Verbindungen der allgemeinen Formel IVa überführt und diese mit komplexen Metallhydriden in einem alkoholischen oder etherischen Lösungsmittel oder in Gemischen derselben zu den entsprechenden 3β-Hydroxy-Verbindungen Va reduziert.

Die Hydroxygruppe der Verbindung III läßt sich sehr gut in eine aliphatische oder aromatische Sulfonsäureestergruppierung überführen, wenn man die Verbindung III bei Temperaturen zwischen -20°C und Raumtemperatur in eine vorher hergestellte Mischung aus dem entsprechenden Sulfonsäurechlorid und einer flüssigen organischen Base eintragt, bis zum vollständigen Umsatz reagieren läßt und dann unter Zusatz von Wasser aufarbeitet. Als Sulfonsäurechlorid wird vorzugsweise ein aromatisches Sulfonsäurechlorid, beispielsweise p-Toluolsulfochlorid und als Base vorzugsweise Pyridin verwendet. Der glatte Reaktionsverlauf ist überraschend, da bekannt ist, daß das 3-Keto-1,5-dien-System sauer oder basisch leicht in das 3-Keto-1,4-dien-System übergeht. Eine derartige Umlagerung wurde bei der Sulfonesterbildung nicht beobachtet.

Die 3-Ketogruppe von Verbindungen der allgemeinen Formel IVa wird durch komplexe Metallhydride wie Natrium-, Calcium- und Lithiumborhydrid oder Lithiumaluminiumhydrid zur 3β-Hydroxygruppe reduziert. Wird dabei unterhalb oder bei Raumtemperatur gearbeitet, erfolgt kein Angriff der gegenüber Reduktionsmitteln empfindlichen primären Sulfonsäureestergruppierung.

Die Reduktion des 3-Ketons wird vorzugsweise mit Calciumborhydrid in Ethanol durchgeführt, hierbei wird reine 3β-Hydroxy-1,5-dien-Verbindung erhalten, die Bildung von 3α-Hydroxy-Verbindung oder von 1,2-gesättigten Produkten unterbleibt praktisch. Eine Rückisomerisierung der 5-Doppelbindung in die 4-Stellung wird nicht beobachtet.

Die Verbindung der allgemeinen Formel Va dient ausschließlich zum Aufbau der für Calcitriol bzw. dessen Abkömmlingen typischen 17-Kohlenstoff-Seitenketten. Dieser Verfahrensteilschritt wird entweder mittels bekannten Methoden oder durch Umsetzung mit einem Nickelaoxacyclohexanon der Formel VI

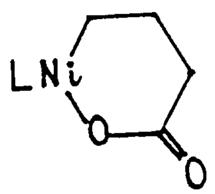

(VI)

durchgeführt.

Zu den bekannten Methoden zählt das im DD-WP 268 956 beschriebene Verfahren, bei welchem eine Verbindung der allgemeinen Formel Va, gegebenenfalls nach Überführung der Sulfonsäureestergruppe in das entsprechende Bromid oder Jodid, mit einer Grignard-Verbindung der Formel

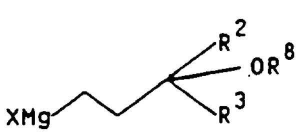

($R^8$= H, Hydroxyschutzgruppe oder MgX, X = Br, Cl)

reagieren gelassen wird. Durch Variation der Kettenlange der verwendeten Grignard-Verbindung sowie der Reste $R^2$ und $R^3$ lassen sich viele verschiedene Verbindungen der Formel VIIa erhalten.

Ein anderes bekanntes Verfahren (DD-A-273 065) ist die Umsetzung der Verbindung Va (nach Austausch der Sulfonestergruppe gegen Brom oder Jod) mit einem Nickelaoxacyclopentanon der Formel

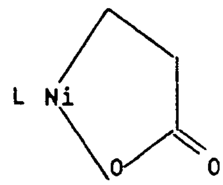

worin L dieselbe Bedeutung wie in der Formel VI hat. Die Umsetzung wird in Gegenwart eines wasserfreien Mangan-II-Salzes in einem organischen Solvens durchgeführt. Dabei entsteht die $C_{25}$-Steroidcarbonsäure

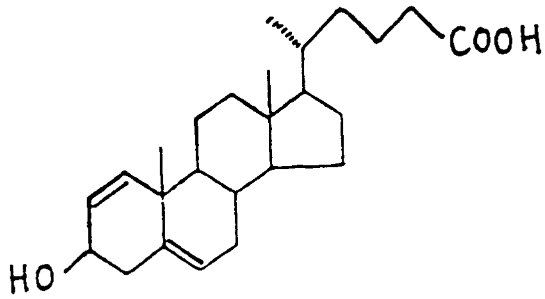

Es wurde nunmehr gefunden daß sich homologe $C_{26}$-Steroidcarbonsäuren der allgemeinen Formel VIIa' durch Umsetzung mit dem entsprechenden, homologen Nickelaoxacyclohexanon der Formel VI gewinnen lassen.

In der Literatur ist die Darstellung von $C_{24}$- und $C_{25}$-Steroidcarbonsäuren in folgenden Patenten und Veröffentlichungen beschrieben:

- US-PS 3 786 062; C.A. 80 (1974) 96229
- Z.obsz. Chim. 45(1975) 925
- Chem. Pharm. Bull. 33 (1985) 878
- DE-OS 2 950 986; C.A. 94 (1981) 4170
- Steroids 13 (1969) 567
- DD-PS 273 065
- Tetrahedron Lett. 31 (1990) 1257.

Die Darstellung von Nickelchelatkomplexen der allgemeinen Formel VI mit z.T. anderen Liganden erfolgte erstmals aus Nickel(O)-Komplexen und Glutarsäureanhydrid (Yamamoto et al., Chem. Lett. 1983, 115). Der entstehende Nikkelacyclus erwies sich jedoch als strukturell nicht einheitlich, vielmehr unterliegt er einer Isomerisierungsreaktion, die unter Ringverengung zu einem verzweigten Chelatring entsprechend der allgemeinen Formel VI'

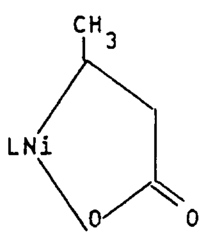

(VI')

führt (Yamamoto et al., Bull.Chem.Soc.Jpn., 57 (1984), 2741). Das Gleichgewicht liegt dabei z.B. bei den Nickelchelatkomplexen mit den oben genannten Liganden zu 80% auf der Seite des Isomeren mit dem verzweigten Chelatring. Die Verwendung dieser Nickelacyclen zur Synthese von $C_{26}$-Steroidcarbonsäuren und zwar in analoger Weise wie in der DD-PS 273 065 beschrieben, führt aufgrund ihrer strukturellen Nichteinheitlichkeit zu einem Produktgemisch, das aus 60% unverzweigter Steroidcarbonsäure und 40% verzweigter Steroidcarbonsäure besteht.

Das ist ein erheblicher Nachteil, da eine nachträgliche Trennung der chemisch sehr ähnlichen Isomeren sehr aufwendig ist und die Gesamtausbeute des Verfahrens stark vermindert wird.

Die Herstellung der $C_{26}$-Steroidcarbonsäure VIIa' erfolgt dadurch, daß $C_{22}$-Steroide der allgemeinen Formel Va nach der Überführung der Sulfonestergruppe in das entsprechende Bromid oder Jodid mit Nickelchelatkomplexen der allgemeinen Formel VI, worin L = 2,2'-Dipyridyl (dipy) oder dessen Alkylsubstitutionsprodukte oder Ethylendiamin bzw. dessen N-alkylierte Derivate bedeutet, in organischen Lösungsmitteln oder Lösungsmittelgemischen umgesetzt werden, dabei für L = dipy oder dessen Alkylsubstitutionsprodukte die Reaktion unter heterogenen Bedingungen und für L = Ethylendiamin bzw. dessen N-alkylierte Derivate in homogener Phase bei gleichzeitiger Einwirkung von Ultraschall durchgeführt wird und die Reaktionsprodukte sauer hydrolysiert werden.

Überraschenderweise führt die erfindungsgemäße Umsetzung der genannten Nickelchelatkomplexe mit den $C_{22}$-Steroiden in Dimethylformamid (DMF) oder in einem Gemisch bestehend aus DMF und einem aromatischen Kohlenwasserstoff zu 100% isomerenreinem unverzweigten $C_{26}$-Steroidcarbonsäuren der allgemeinen Formel VIIa'.
Entscheidend für den vorteilhaften Verlauf der Reaktion ist die heterogene Reaktionsführung im Fall des den Liganden dipy bzw. seine alkylsubstituierten Derivate enthaltenden schwerlöslichen Nickelchelatkomplexes bzw. die gleichzeitige Einwirkung von Ultraschall im Fall des Komplexes mit Ethylendiamin bzw. dessen Alkylsubstitutionsprodukte als Ligand.

Die Reaktionstemperatur kann zwischen Raumtemperatur und 55°C gewählt werden, die Reaktionszeit liegt zwischen 5 Stunden und 48 Stunden. Vorzugsweise werden die Steroidhalogenide bei 30°C umgesetzt. Für die Umsetzung von 1 Mol Steroidhalogenid werden 1,5 Mol bis 2,5 Mol Nickelchelatkomplex eingesetzt.
Zur Erhöhung der Reaktionsgeschwindigkeit und Ausbeute erweist sich der Zusatz von wasserfreien Mangan(II)-Salzen wie Manganiodid, Manganbromid oder Manganacetat als günstig. Vorzugsweise wird ein Mol Manganiodid pro Mol Steroidhalogenid eingesetzt. Nach saurer Hydrolyse werden die $C_{26}$-Carbonsäuren in hohen Ausbeuten und isomerenrein erhalten. Die erhaltenen $C_{26}$-Carbonsäuren können nach an sich bekannten Verfahren der organischen Chemie derivatisiert werden (Ester, Amide, Säurechloride usw.)

Der Vorteil des Verfahrens besteht im erstmals möglichen Zugang zu unverzweigten $C_{26}$-Steroidcarbonsäuren aus gut zugänglichen $C_{22}$-Steroiden und Nickelchelatkomplexen der allgemeinen Formel VI in einem Verfahrensschritt und in hohen Ausbeuten und mit 100%iger Isomerenreinheit.

Vor der Oxidation der Steroidcarbonsäuren der allgemeinen Formel VIIa' bzw. der mit dem Nickelaoxacyclopentanon erhaltenen $C_{25}$-Steroidcarbonsäuren werden in diese noch die Reste $R^2$ und $R^3$ durch doppelte Grignardierung mit einer Verbindung $R^{23}$MgHal ($R^{23} = R^2 = R^3$; Hal = Cl, Br, J) eingeführt.

Im nächsten Reaktionsschritt werden die Verbindungen der allgemeinen Formeln VIIa bzw. VIIa' (nach doppelter Grignardierung) selektiv in 7-Stellung oxidiert und anschließend die 1,2-Doppelbindung epoxidiert. Der stereochemische Verlauf der Epoxidierung hängt dabei sehr stark vom jeweils vorhandenen Substituenten $R^1$ am Sauerstoffatom des 3-Kohlenstoffatoms ab.

Die Einführung einer 7-Ketogruppe in $\Delta^5$-Steroide mit Hilfe von Chrom(VI)Verbindungen ist bereits bekannt, während die Oxidation von 3β-substituierten $\Delta^{1,5}$-Steroiden bisher nicht beschrieben ist.
Es wurde gefunden, daß die gut zugänglichen 3β-substituierten 1,5-Dien-Steroide (Shapiro, E. et al., Steroids 8 (1966) 461) der allgemeinen Formeln VIIa bzw. VIIa' sich überraschenderweise mit Chrom(VI)-Verbindungen in organischen Lösungsmitteln zu den entsprechenden 7-Ketonen der allgemeinen Formel VIIIa oxidieren lassen.
Die Oxidation läßt sich mit den in der Literatur für Allyloxidationen beschriebenen Systemen Natriumchromat in Eisessig/Acetanhydrid (US 2 505 646), Chromtrioxid in Pyridin (Dauben, W.G., M. Lorber und D.S. Fullerton, J. Org. Chem. 34 (1969) 3587; Collins, J.C., W.W. Hess und F.J. Frank, Tetr. Lett. 1968, 3363). Chromtrioxid und Dimethylpyrazol oder Pyrazol in Dichlormethan (Salmond, W.G., M.A. Barta und J.L. Havens, J. Org. Chem. 43 (1978) 2057; US 4 006 172), tert.-Butylchromat in Tetrachlormethan (Heusler, K. und A. Wettstein, Helv.Chim. Acta 35(1952) 284), Chromtrioxid unter Phasentransferbedingungen (Singh, C., Indian J. Chem. Sect. B 24B (1985) 300) oder tert.-Butylhydroperoxid in Gegenwart katalytischer Mengen Pyridiniumchlorochromats (N. Chidambaran et al., J. Org. Chem. 52 (1987), 5048) durchführen.

Überraschenderweise findet keine oder nur untergeordnete Reaktion am 3β-substituierten $\Delta^1$-Allyl-System statt.
Die Reaktionstemperaturen liegen zwischen -50°C und +40°C in Abhängigkeit von der angewandten Methode.
Die Aufarbeitung erfolgt in üblicher Weise durch Zusatz von Wasser und Trennung der Chromsalze vom Steroid.
Der folgende Reaktionsschritt dient der Einführung einer 1,2-Epoxygruppe in die Verbindungen der allgemeinen Formel VIIIa. Die Epoxidation wird mit organischen Persäuren in organischen Lösungsmitteln durchgeführt. Vorhandene Schutzgruppen werden anschließend gegebenenfalls nach gängigen Verfahren abgespalten.
Ein Silylrest als $R^1$ dirigiert die eintretende Epoxygruppe vornehmlich in die 1,2α-Stellung, Epoxidierung der entsprechenden 3-Acyloxyverbindung führt im wesentlichen zum 1,2β-Epoxid. In beiden Fällen erfolgt die Epoxidation vorzugsweise mit 1,5 bis 4 Mol organischer Persaure pro Mol Steroid. Als Persäuren werden z.B. m-Chlorperbenzoesäure, Perbenzoesäure, Monoperphthalsäure oder m-Dinitroperbenzoesäure in Ether, Toluol, Benzol, Chloroform oder Dichlormethan bei Raumtemperatur oder bei Temperaturen bis +50°C verwendet. Unter diesen Bedingungen wird kein oder nur unwesentlicher Angriff an der 5-Doppelbindung beobachtet.

Nach vollständigem Umsatz wird basisch aufgearbeitet. Das als Hauptprodukt entstandene 1α,2α- bzw. 1β,2β-Epoxid wird durch Chromatographie oder Umkristallisation vom jeweils mitentstandenen, anderen Epoxid befreit.
Die Spaltung der 3β-Silylethergruppe zu den entsprechenden 3β-Hydroxyverbindungen kann sauer oder mit Fluoridionen erfolgen, ohne daß Eliminierung oder Epoxidöffnung stattfindet.
Alternativ zur direkten Epoxidierung einer Verbindung der allgemeinen Formel VIIIa, worin $R^1$ einen Acyl- oder Aroylrest bedeutet, kann das 1,2β-Epoxid der allgemeinen Formel IXaβ

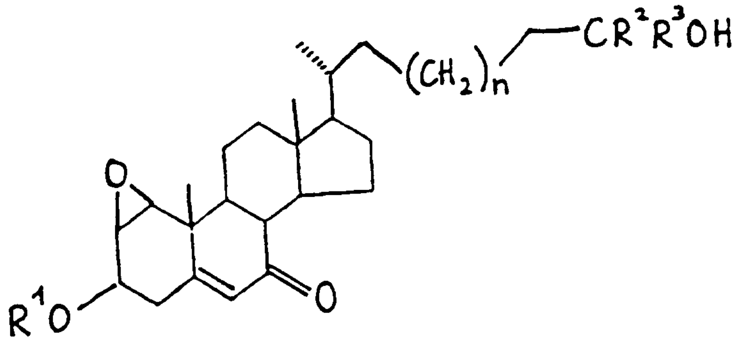

(IXaβ)

auch über ein 2β-O-substituiertes 1α-Halogen-3β-hydroxy-$\Delta^5$-7-keto-Steroid der allgemeinen Formel XI

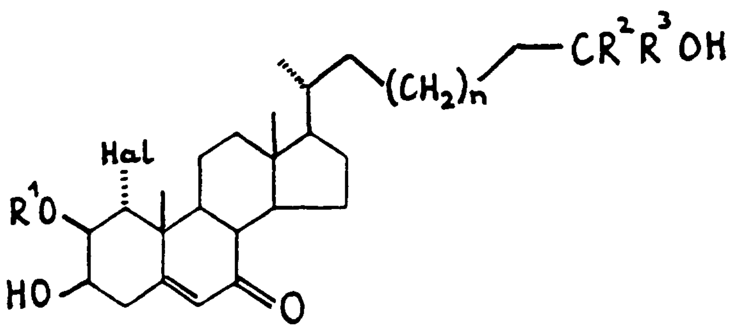

(XI)

erhalten werden.

Die Verbindungen der allgemeinen Formel XI entstehen bei der Umsetzung der Verbindungen der allgemeinen Formel VIIIa mit Verbindungen, die positiviertes Halogen, insbesondere positiviertes Brom, enthalten, in organischen Lösungsmitteln unter Wasserzusatz.

Es ist bekannt, daß Bromhydrine aus Olefinen mit N-Bromacetamid oder N-Bromsuccinimid oder anderen Verbindungen, die positiviertes Brom enthalten, in wäßrigen organischen Lösungsmitteln mit oder ohne Zusatz von katalytischen Mengen Säure hergestellt werden können (Houben-Weyl, Band V/4, S. 139, Georg Thieme Verlag, Stuttgart 1960; H.B. Henbest, R.A.L. Wilson, J. Chem. Soc. 1959, 4136, E. Glotter, P. Krinsky, J. Chem. Soc. Perkin I 1978, 408; Fieser, Fieser, Reagents for Organic Syntheses, Bde. 1 bis 11, Verlag J. Wiley, New York).

Die Verbindungen der allgemeinen Formel XI werden durch Behandlung mit schwachen Basen in die entsprechenden 1ß,2β-Epoxy-Steroide überführt. Dabei wandert der Rest $R^1$ von der 2- in die 3-Position des Steroidgerüstes zurück.
Die Umsetzung der Verbindungen der allgemeinen Formel VIIIa zu den Verbindungen der allgemeinen Formel XI läßt sich mit N-Bromacetamid oder N-Bromsuccinimid in Dioxan/Wasser, Aceton/Wasser, tert.Butanol/Wasser, Pyridin/Benzol/Wasser mit oder ohne Zusatz von katalytischen Mengen Säure wie p-Toluolsulfonsäure, Schwefelsäure, Eisessig oder Perchlorsäure erreichen. Als Lösungsmittel können weiterhin Dimethoxyethan/Wasser, Tetrahydrofuran/Wasser und Dimethylsulfoxid/Wasser verwendet werden.

Als positive Bromquelle können auch Verbindungen wie Dibromhydantoin dienen. Bevorzugt wird die Reaktion in Tetrahydrofuran/Wasser mit N-Bromacetamid bei Raumtemperatur durchgeführt. Es wird ein 4- bis 15-facher Überschuß an Bromierungsmittel verwendet. Die Reaktionstemperatur kann zwischen -5°C und +30°C variiert werden. Der vollständige Umsatz wird durch Dünnschichtchromatographie ermittelt. Die Aufarbeitung erfolgt in üblicher Weise durch Zusatz von Wasser. Dabei fallen die gewünschten Reaktionsprodukte in guten Ausbeuten und mit hoher Reinheit an.

Der praktisch vollständige Angriff des elektrophilen Broms von der α-Seite ist im Hinblick auf den bekannten, bevorzugt von der β-Seite erfolgenden Angriff der elektrophilen Persäuren als überraschend zu werten.
Eine Reaktion an der 5-Doppelbindung findet unter den angegebenen Bedingungen nicht statt.

Die 1,2α- und 1,2β-Epoxy-7-Keto-Steroide der allgemeinen Formel IXa werden dann mit (4-Methylphenylsulfonyl)hydrazid in einem organischen protischen (z.B. niederer Alkohol), dipolar-aprotischen (z.B. Ether wie Tetrahydrofuran) oder unpolaren Solvens (z.B. Toluol) unter Erwärmen in ihre entsprechenden 7-(4-Methylphenylsulfonyl)hydrazone Xa überführt und diese mit Lithiumhydrid unter anaeroben Bedingungen in einem geeigneten Ether (z.B. Tetrahydrofuran) oder unpolaren Lösungsmittel (z.B. Toluol) unter Erhitzen zu den letztendlich gewünschten Produkten der allgemeinen Formel Ia mit einem konjugierten 5(6), 7(8)-Diensystem umgesetzt.

Sollen nach dem erfindungsgemäßen Verfahren Ausgangsverbindungen der allgemeinen Formel Ib zur Herstellung von Calcitriol-Abkömmlingen erhalten werden, muß die vorstehend beschriebene Reaktionssequenz modifiziert werden, und zwar so, daß erst im letzten Reaktionsschritt der zum Seitenkettenaufbau taugliche $C_{22}$-Aldehyd der allgemeinen Formel Ib (bzw. dessen $C_{23}$-Homologes) aufgebaut wird.

Diese Modifikation betrifft aber nur die an der $C_{17}$-Seitenkette erforderlichen Reaktionen. Die am Steroidgrundgerüst durchzuführenden Reaktionsschritte zur Einführung des 5(6), 7(8)-Diensystems in Gegenwart einer 1,2α-Epoxygruppe sind identisch wie die vorstehend unter III→IVa→Va→VIIa/VIIa'→VIIIa→IXa→Xa→Ia beschriebenen. In der Reaktionssequenz III→IVb Vb→VIII→IXb→Xb→Ib wird der in vorstehender Syntheseroute enthaltene und dem Seitenkettenaufbau dienende Schritt Va→VIIa bzw. VIIa' übersprungen, da die $C_{17}$-Seitenkette erst ausgehend vom Aldehyd Ib aufgebaut wird.

Ebenfalls ausgehend von der Verbindung der Formel III wird die Hydroxygruppe in eine leicht abspaltbare Schutzgruppe $R^X$ überführt (Verbindung der allgemeinen Formel IVb); als Beispiele seien niedere Alkylether, das Formiat sowie Tetrahydropyranylether genannt.
Reduktion zur 3-Hydroxyverbindung der allgemeinen Formel Vb wird wie die Reduktion IVa→Va, beispielsweise mit Calciumborhydrid als Reduktionsmittel, durchgeführt. Vor der Allyloxidation in 7-Position analog VIIa/VIIa'→ VIIIa wird die 3-Hydroxygruppe mit einem Alkoxycarbonylchlorid der allgemeinen Formel R"OC(O)Cl (R" = gerad- oder verzweigtkettiger Alkylrest mit 1 bis 6 Kohlenstoffatomen) in Gegenwart einer Base verestert.
Epoxidierung zu den 1,2α-Epoxiden der allgemeinen Formel IXb und Bildung des 7-Phenylhydrazons Xb geht ebenfalls analog zur Umsetzung VIIIa→IXa→Xa vonstatten.

Selektive Abspaltung der leicht abspaltbaren Schutzgruppe $R^X$ unter milden Bedingungen und Oxidation der freigesetzten 20-Hydroxymethylverbindung der allgemeinen Formel Ib" ergibt den letztendlich gewünschten Aldehyd der allgemeinen Formel Ib.

Als Oxidationsmittel kommen beispielsweise Pyridiniumchlorochromat, Pyridiniumdichromat oder Dimethylsulfoxid infrage.

Gewünschtenfalls kann die $C_{17}$-Seitenkette der Hydroxymethylverbindung Ib" oder des Aldehyds Ib nach gängigen Verfahren homologisiert werden.

Die nachfolgenden Beispiele dienen der näheren Erläuterung der Erfindung.

## BEISPIELE

(20S)-20-Hydroxymethyl-1,5-pregnadien-3-on

Kaliumbutan-2-olat, hergestellt aus 17,5 g Kaliumhydroxid und 250 ml Butan-2-ol, wird mit einer Lösung von 10 g (20S)-20-Hydroxymethyl-1,4-pregnadien-3-on in 150 ml trockenem Tetrahydrofuran unter Schutzgas versetzt. Die Suspension wird 3 Stunden bei Raumtemperatur gerührt. Danach wird das Gemisch bei -10°C in eine Losung, bestehend aus 200 ml einer mit Ammoniumchlorid und Kohlendioxid gesättigten wäßrigen Lösung und 8 ml halbkonzentrierter Salzsäure, unter Rühren eingetragen, wobei die Temperatur ansteigen kann. Der pH-Wert wird auf 4 eingestellt, nach 60 Minuten wird abgesaugt, mit Wasser neutral gewaschen und getrocknet.
Ausbeute: 9.9 g Fp.: 158-163,5°C (Zers.), Umkristallisation aus Methanol erhöht den Schmelzpunkt auf 168-172°C.

(20S)-20-Tosyloxymethyl-1,5-pregnadien-3-on

56 g p-Toluolsulfochlorid und 200 ml trockenes Pyridin werden 20 Minuten bei -10°C gerührt. Dazu werden 40 g (20S)-20-Hydroxymethyl-1,5-pregnadien-3-on gegeben. Nach 4stündigem Rühren bei 0°C wird das Reaktionsgemisch in 2 Liter Eiswasser getropft und noch eine Stunde nachgerührt. Das Produkt wird abgesaugt, mit Wasser gewaschen und getrocknet.
Ausbeute: 58 g Fp.: 116-122° Umkristallisation aus Aceton erhöht den Schmelzpunkt auf 125-128°C. $[\alpha]_D^{20}$ + 37° (o = 1, $CHCl_3$)

(20S)-20-Tosyloxymethyl-3β-hydroxy-1,5-pregnadien

Eine Lösung von 53 g wasserfreiem Calciumchlorid in 1 l abs. Ethanol wird bei -30°C unter Rühren zu einer gekühlten Lösung von 32 g Natriumborhydrid in 1 l abs. Ethanol getropft. Die so erhaltene Calciumborhydridlösung wird portionsweise bei -20°C unter Rühren zu einer Suspension aus 50 g (20S)-20-Tosyloxymethyl-1,5-pregnadien-3-on in 660 ml abs. Ethanol gegeben. Nach 3 Stunden wird 1 l Aceton zugetropft, wobei die Temperatur auf 0°C ansteigt. Nach 30 Minuten Rühren werden 2 l Wasser zugetropft und nach weiteren 30 Minuten Rühren setzt man 100 ml halbkonzentrierte Salzsäure zu. Die Suspension wird abgekühlt und nach mehreren Stunden im Vakuum auf ca. 1 l Volumen eingeengt. Danach wird mit 4 l Wasser versetzt und wieder abgekühlt. Nach mehreren Stunden wird das Produkt abgesaugt, mit Wasser gewaschen und getrocknet.
Ausbeute: 51 g Fp.: 77-83°C. Zur Reinigung wird vorsichtig aus Methanol umkristallisiert. Man erhält 35 g feine, weiße Kristalle. Fp.: 85-88°C.
$[\alpha]_D^{20}$ -17° (c = 1, $CHCl_3$)

1,5-Cholestadien-3β,25-diol

In einer trockenen Apparatur legt man unter Argondruck 38 ml einer aus 650 mg Mg, 37 ml trockenem THF und 3,11 g 2-Methyl-2-trimethylsilyloxy-4-chlorbutan frisch zubereiteten Grignardlösung vor und kühlt auf 0°C. Dann wird tropfenweise mit 2 ml einer frisch aus 48 mg trockenem Lithiumchlorid, 77,6 mg trockenem Kupfer(II)chlorid und 5,6 ml THF zubereiteten Dilithiumtetrachlorocupratlösung versetzt. Nach 10 Minuten gibt man innerhalb von 2 Minuten eine Lösung von 810 mg 3β-tert.-Butyl-dimethylsilyloxy-22-tosyloxy-23,24-bisnorchola-1,5-dien in 11 ml THF zu, rührt 2 Stunden bei 0°C und läßt dann auf Raumtemperatur erwärmen. Das Gemisch wird über Nacht stehen gelassen, filtriert, mit ges. $NH_4Cl$-Lösung auf etwa 240 ml aufgefüllt; nach Phasentrennung, Nachextraktion der wäßrigen Phase mit Benzol, Neutralwaschen, Trocknen mit $Na_2SO_4$, Filtrieren und Einengen am Vakuumrotationsverdampfer erhält man eine leicht gelbliche kristalline Substanz. Der Rückstand wird mit 15 ml Aceton versetzt und die Suspension zum dicken Kristallbrei eingeengt. Man saugt dann kalt ab, wäscht mit wenig kaltem Aceton und erhält 740 mg 3β-tert-Butyl-dimethylsilyloxy-25-trimethylsilyloxy-cholesta-1,5-dien (weiße, blätterförmige Kristalle).
Fp.: 142-144°C
$[\alpha]_D^{20}$ (c= 1,$CHCl_3$) +25,5°

Zur Abspaltung der Schutzgruppen wird in Tetrahydrofuran aufgenommen und die Lösung bei Raumtemperatur mit verdünnter Salzsäure versetzt. Nach zwei Stunden wird durch Zusatz von Wasser, Extraktion mit Methylenchlorid, Neutralwaschen und Einengen am Vakuumrotationsverdampfer aufgearbeitet. Der Rückstand ergibt aus Aceton 1,5-Cholestadien-3β,25-diol in Form weißer Kristalle.
Fp.: 153-156°C
$[\alpha]_D^{20}$ (c= 1,$CHCl_3$) -5°

3β-Hydroxy-24a,24b-bishomo-5-cholen-24b-säure und deren Methylester

800 mg (1,81 mmol) 3β-Hydroxy-20-iodmethyl-5-pregnen werden mit 500 mg (1,62 mmol) Mangan(II)-iodid und 1010 mg (4,18 mmol) Nickelchelatkomplex der Formel VI bzw. VI' (L = N,N,N',N'-Tetramethylethylendiamin) in 30 ml Dimethylformamid gelöst und 2 Stunden im Ultraschallbad zur Reaktion gebracht. Der Verlauf der Reaktion wird dünnschichtchromatographisch verfolgt. Zur Aufarbeitung wird das Lösungsmittel im Vakuum abgezogen, der Rückstand mit Wasser und verdünnter Salzsäure aufgenommen, die wäßrige Phase mehrmals mit Chloroform extrahiert, die organische Phase mit Wasser, Thiosulfatlösung und nochmals mit Wasser gewaschen und über Natriumsulfat getrocknet. Das Extraktionsmittel wird vollständig abdestilliert und der Rückstand aus Methanol umkristallisiert.
Die Veresterung der Säure erfolgt nach der Vorschrift in Ind. J. Chem. 22B (1983), 505 mit Methanol und Trimethylchlorsilan; Ausbeute nach Umkristallisation aus Aceton: 430 mg
Fp.: 115-119°C
$[\alpha]_D^{20}$ (c= 1,$CHCl_3$) -35°

3β-Acetoxy-25-hydroxy-cholesta-1,5-dien

20 g 3β,25-Dihydroxycholesta-1,5-dien werden in einem 75 ml-Sulfierkolben unter Feuchtigkeitsausschluß in 90 ml Pyridin gelöst, auf eine Temperatur von - 5°C gekühlt und bei dieser Temperatur unter Rühren mit 90 ml Essigsäureanhydrid so versetzt, daß die Temperatur + 5°C nicht- übersteigt. Bei dieser Temperatur (0 - 5°C) wird 20 Minuten gerührt. Anschließend läßt man auf Raumtemperatur erwärmen. Die Umsetzung ist nach ca. 3 Stunden Rühren bei Raumtemperatur beendet. Zur Aufarbeitung wird der Ansatz unter intensivem Durchschütteln auf 1l Eiswasser gegeben. Das Endprodukt kristallisiert sofort aus und wird, nachdem das Eis geschmolzen ist, über eine G 3-Fritte abgesaugt, mit ca.

1l Wasser gewaschen und trockengezogen.
Fp.: 119-121°C

3β-Acetoxy-25-hydroxy-cholesta-1,5-dien-7-on

a) 0,2 g (0,76 mmol) geschmolzenes wasserfreies Natriumchromat werden mit 0,2 g Kieselgel (Merck 60, 0,2 - 0,5 mm) unter Verreiben innig gemischt und zu einer Lösung von 10 g (22,6 mmol) 3β-Acetoxy-25-hydroxycholesta-1,5-dien in 80 ml Benzol zugegeben. Anschließend versetzt man mit 12 ml (etwa 96 mmol) ca. 80%igem t-Butylhydroperoxid und rührt 4 bis 5 Stunden bei 50°C. Nach dem Ende der Reaktion (DC-Kontrolle, Kieselgel Merck, Laufmittel Benzen / Aceton 9:1) wird mit Ether (400 ml) verdünnt, filtriert, das Filtrat eingeengt und der Rückstand durch Säulenchromatographie (Kieselgel Merck 60, 0,2-0,5 mm, 100 g) gereinigt. Dazu wird der Rückstand in 50 ml Essigsäureethylester gelost, an 10 g Kieselgel gebunden und am Rotationsverdampfer das Lösungsmittel abdestilliert. Das Kieselgel wird auf die bereits gefüllte Säule gegeben. Anschließend eluiert man mit Hexan / Essigsäureethylester mit ansteigenden Mengen Ester bis zum Verhältnis 60 : 40 (1,21 Hexan, 400 ml Essigester über Molsieb 4Å getrocknet). Nach Einengen erhält man 9,6 g (94 %) gereinigtes Rohprodukt, das unter Hexan fest und ohne weitere Reinigung in die nächste Stufe eingesetzt wird. Eine weitergehende Reinigung kann durch Säulenchromatographie an Kieselgel Merck 60 (0,063 -0,2 mm) unter Druck (Elution mit Hexan / Essigsäureethylester bis zum Verhältnis 60 : 40) erreicht werden. Ausbeute: 7,6 g (74 %), (UV($CH_3OH$)$\lambda_{max}$ = 233 nm, ε = 10541). Für analytische Zwecke wurde eine Probe durch wiederholte Druckchromatographie bzw. durch Umkristallisation aus Hexan/Ether 1:1 noch weiter gereinigt.
Fp. 129 - 131°C, $[\alpha]_D^{20}$ : -36,8° ($CHCl_3$); - 26,9° ($CH_3OH$) MS, m/e: 456 ($M^+$), 438 ($M^+$), 438 ($M^+$-$H_2O$), 396 ($M^+$-$CH_3COOH$), 378 ($M^+$-$H_2O$-$CH_3COOH$)

b) 0,79 g Pyridin werden unter Rühren bei -30°C mit einer Lösung von 1 g Chromtrioxid in 20 ml Dichlormethan versetzt. Nach 10 Minuten werden 200 mg 3β-Acetoxy-25-hydroxy-cholesta-1,5-dien zugegeben. Das Reaktionsgemisch wird unter Rühren auf Raumtemperatur gebracht. Die Reaktion ist nach 3 bis 4 Stunden beendet. Nach Zugabe von gesättigter wäßriger Natriumsulfitlösung und Ansäuern mit 10%iger Salzsäure wird die organische Phase abgetrennt und die wäßrige Phase zweimal mit Ether nachextrahiert. Die organischen Phasen werden neutral gewaschen, über $Na_2SO_4$ getrocknet und im Vakuum eingedampft. Das Rohprodukt wird durch Säulenchromatographie an Kieselgel (Merck) mit Benzin/Essigsäureethylester 75:25 v/v gereinigt.
Ausbeute: 185 mg
Fp.: 136°C (Ether/Hexan)

c) Eine Lösung von 1 g Chromtrioxid in 15 ml abs. Dichlormethan werden bei -30°C unter Rühren mit 1 g 3,5-Dimethylpyrazol versetzt. Zu dieser Lösung werden 170 mg 3β-Acetoxy-25-hydroxy-cholesta-1,5-dien gegeben. Die Umsetzung ist nach 4 Stunden Rühren bei -20°C beendet. Die Reaktionslösung wird mit 10 ml gesättigter Natriumbisulfitlösung versetzt und die wäßrige Phase durch Zugabe von 10%iger Salzsäure auf pH 5 bis 6 eingestellt. Die organische Phase wird abgetrennt, neutral gewaschen und getrocknet. Die Reinigung des Rohprodukts erfolgt säulenchromatographisch wie unter a) beschrieben.
Ausbeute: 155 mg
Fp.: 136°C (Ether/Hexan)

3β,25-Dihydroxy-cholesta-1,5-dien-7-on

In einem 500 ml-Einhalskolben werden 10 g 3β-Acetoxy-25-hydroxy-cholesta-1,5-dien-7-on in 80 ml Dioxan gelöst und mit 80 ml Methanol versetzt. Danach wird eine Lösung von 10 g Kaliumcarbonat in 40 ml destilliertem Wasser zugegeben, der Kolben geschüttelt und nachfolgend auf dem Wasserbad auf 40°C erwärmt. Nach 60 Minuten bei 40°C wird die Reaktion dünnschichtchromatographisch auf Vollständigkeit geprüft. Zur Aufarbeitung wird das Reaktionsgemisch am Rotationsverdampfer bis zur beginnenden Kristallisation eingedampft und danach in 500 ml Wasser gegossen. Das amorphe Festprodukt saugt man ab, wäscht drei- bis viermal mit Wasser und trocknet im Ölpumpenvakuum bei 50°C. Zuletzt wird aus 50 ml Diethylether umkristallisiert. Das Kristallisat wird abgesaugt, mit wenig kaltem Diethylether gewaschen und im Ölpumpenvakuum getrocknet. Man erhält 7,7 g (84,8 %) Produkt in Form kristalliner Blättchen.
Fp.: 173-174°C, $[\alpha]_D^{20}$ (c=1,$CH_3OH$)-54°, UV($CH_3OH$):$\lambda_{max}$=236 nm, ε =11360

3β-tert.-Butyldimethylsilyloxy-25-hydroxy-cholesta-1,5-dien-7-on

a) In einem 100 ml-Einhalskolben werden 8 g 3β,25-Dihydroxy-cholesta-1,5-dion-7-on in 30 ml Dimethylformamid gelöst und mit 8 g Imidazol versetzt. Anschließend gibt man 8 g t-Butyldimethylsilylchlorid zu. Nach 5-10 Minuten

setzt Kristallisation ein. Man rührt noch 30 Minuten, löst den Kristallbrei in einem Gemisch von 20 ml Diethylether und 200 ml Wasser und überführt in einen Scheidetrichter. Die Phasen werden nach kräftigem Schütteln getrennt. Die etherische Phase wäscht man mehrmals mit je 50 ml Wasser und trocknet danach mit Natriumsulfat. Das Lösungsmittel wird im Vakuum (Rotationsverdampfer) abdestilliert. Das dabei auskristallisierende Rohprodukt wird in 100 ml Chloroform gelöst, an 20 g Kieselgel gebunden und das Lösungsmittel am Rotationsverdampfer abdestilliert. Das Adsorbat gibt man auf eine kurze Säule auf (50 g Kieselgel, Durchmesser: 5 cm) und eluiert mit Benzol:Chloroform 1:1 (l l). Das Lösungsmittel wird im Rotationsverdampfer abdestilliert und man erhält 9,6 g kristallines 3β-(t-Butyldimethylsilyloxy)-25-hydroxycholesta-1,5-dien-7-on. Eine weitere Reinigung kann durch Kristallisation aus Methanol/Hexan erfolgen.
Fp.: 188-191°C, $[\alpha]_D^{20}$ (Ethanol)-19°, UV($CH_3OH$):$\lambda_{max}$=236 nm, ε =12400

b) Eine Suspension von 5,84 g Chromtrioxid in 51,5 ml abs. Dichlormethan wird bei -30°C unter Rühren mit 5,84 g 3,5-Dimethylpyrazol versetzt. Zu dieser Lösung wird nach 20 Minuten eine Lösung von 1,73 g 3β-tert.-Butyldimethylsilyloxy-25-hydroxy-cholesta-1,5-dien in 34,5 ml abs. Dichlormethan innerhalb von 5 Minuten bei -25°C zugegeben. Nach einer Stunde, bei der die Temperatur zwischen -20°C und 0°C liegt, wird mit 180 ml verdünnter Salzsäure (1:1) und mit 540 ml gesättigter Natriumbisulfitlösung versetzt. Die organische Phase wird abgetrennt, die wäßrige Phase wird zweimal mit je 200 ml Dichlormethan extrahiert. Nach Vereinigung der organischen Phasen wird fünfmal mit je 150 ml halbkonzentrierter Salzsäure, zweimal mit je 130 ml gesättigter Natriumcarbonatlösung und zweimal mit Wasser gewaschen, getrocknet und im Vakuum eingeengt. Der Rückstand wird an 40 g Kieselgel 60 (Fa Merck) mit Benzol/Essigester 95,5 v/v und 90:10 v/v chromatographiert.
Ausbeute: 1,22 g
Fp.: 190-192°C

3β-Acetoxy-25-hydroxy-1α,2α-epoxy-cholest-5-en-7-on

a) 3β-tert.-Butyldimethylsilyloxy-1α,2α-epoxy-25-hydroxy-cholest-5-en-7-on
530 mg 3β-tert.-Butyldimethylsilyloxy-25-hydroxy-cholesta-1,5-dien-7-on, gelöst in 30 ml abs. Dichlormethan, werden mit 460 mg m-Chlorperbenzoesäure versetzt und auf 40°C erwärmt. Nach 4 Stunden wird im Vakuum zur Trockne eingeengt, der Rückstand in Ether gelöst und mit gesättigter $NaHCO_3$-Lösung extrahiert.
Die organische Phase wird mit Wasser gewaschen, getrocknet und im Vakuum eingedampft. Der Rückstand (502 mg) wird an Kieselgel 60 (Fa. Merck) mit Benzol/Essigester 90:10 v/v chromatographiert.
Ausbeute: 320 mg
Fp.: 191-194°C ($CHCl_3$/Hexan) UV:$\lambda_{max}$ = 235 nm, ε =11100 ($CH_3OH$, c=0,68)

b) 3β,25-Dihydroxy-1α,2α-epoxy-cholest-5-en-7-on
In einem 500 ml-Einhalskolben werden 5,5 g 3β-tert.-Butyldimethylsilyloxy-1α,2α-oxido-25-hydroxy-cholest-5-en-7-on in 100 ml Tetrahydrofuran gelöst und mit 100 ml Eisessig sowie unter intensivem Schütteln mit 50 ml Wasser versetzt. Der Kolben wird mit einem Rückflußkühler versehen und das Reaktionsgemisch wird im Wasserbad auf 60°C erwärmt. Die Reaktion ist nach ca. 3 Tagen beendet (DC-Kontrolle), gegebenenfalls wird die Reaktionszeit verlängert. Nach vollständigem Umsatz kühlt man auf Raumtemperatur ab und dampft am Rotationsverdampfer bis zur beginnenden Kristallisation ein. Anschließend werden 200 ml Wasser zugegeben und der Niederschlag abgesaugt (G 3-Fritte). Mit destilliertem Wasser wird bis zur Neutralität gewaschen (Kontrolle mit Indikatorpapier). Den amorphen Filterrückstand löst man in Chloroform und trocknet die Losung mit Natriumsulfat. Nach dem Absaugen des Natriumsulfats wird das Chloroform am Rotationsverdampfer abdestilliert. Das so erhaltene Rohprodukt (3,7 g Schaum) wird im folgenden Verfahren ohne weitere Reinigung acetyliert (siehe folgendes Beispiel).

c) 3β-Acetoxy-25-hydroxy-1α,2α-epoxy-cholest-5-en-7-on
In einem 250 ml-Einhalskolben werden 4,3 g 3β,25-Dihydroxy-1α,2α-epoxycholest-5-en-7-on in 50 ml frisch destilliertem Pyridin gelöst. Die Lösung kühlt man auf +10°C und versetzt portionsweise mit 50 ml Essigsäureanhydrid (Erwärmung des Kolbens vermeiden). Der Kolben wird verschlossen und man läßt ca. 2 Stunden bei Raumtemperatur stehen (DC-Kontrolle auf Vollständigkeit der Reaktion).
Zur Aufarbeitung wird das Reaktionsgemisch unter intensivem Rühren langsam in 300 ml Wasser gegossen. Anschließend wird noch 20 Minuten nachgerührt. Man laßt den feinkristallinen Niederschlag absitzen und filtriert ihn über eine Fritte (G 3) ab. Der Filterrückstand wird solange mit destilliertem Wasser gewaschen, bis dieser pyridin- und essigsäureanhydridfrei ist (Geruchsprobe). Das Rohprodukt (ca. 5 g) trocknet man im Vakuumexsikkator über Phosphorpentoxid.
2,5 g des Rohprodukts (Gemisch aus ca. 90% α-Epoxid und 10% β-Epoxid) werden in 50 ml Chloroform gelost, mit 6 g Kieselgel Merck 60 versetzt. Das Chloroform wird am Rotationsverdampfer aodestilliert. Das erhaltene Absorbat gibt man auf eine Säule auf (100 g Kieselgel Merck 60, Durchmesser: 2 cm, Länge: 100 cm) und eluiert mit Benzol/Essigsäureethylester 7:3 (Fraktionsgröße: 15 ml). Die reines Epoxid enthaltenden Fraktionen werden vereinigt, bei 40°C am Rotationsverdampfer eingedampft und der Rückstand am Ölpumpenvakuum bei Raumtemperatur bis zur Gewichtskonstanz getrocknet (Ausbeute 1,7 g).
Durch Kristallisation aus Ethylacetat/Hexan erhält man analysenreines Produkt.
Fp.: 118-120°C, $[\alpha]_D^{20}$ ($CH_3OH$)-26,8° UV($CH_3OH$):$\lambda_{max}$ = 232 nm, $\varepsilon$ = 11234

3β-Acetoxy-25-hydroxy-1α,2α-epoxy-cholest-5-en-7-(4-methylphenylsulfonyl)hydrazon
Umsetzung von 3β-Acetoxy-25-hydroxy-1α,2α-epoxy-cholest-5-en-7-(4-methyl-phenyl-sulfonyl)-hydrazon

Die Umsetzung mit (4-Methyl-phenyl-sulfonyl)-hydrazid erfolgt in einem trockenen 100 ml-Dreihalskolben mit Rückflußkühler unter anaeroben Bedingungen (schwacher Argonstrom). 4,8 g 3β-Acetoxy-25-hydroxy-1α,2α-epoxy-cholest-5-en-7-on werden in 100 ml abs. Tetrahydrofuran gelöst und mit 4 g (4-Methyl-phenyl-sulfonyl)-hydrazid versetzt. Der Kolben wird mit Aluminiumfolie vor Lichteinfluß geschützt. Anschließend kocht man das Reaktionsgemisch 6 Stunden am Rückfluß und läßt danach 12 Stunden bei Raumtemperatur stehen. Sollte die Reaktion unvollständig sein (DC-Kontrolle), wird nochmals 1 g (4-Methyl-phenyl-sulfonyl)-hydrazid zugegeben, das Reaktionsgemisch weitere 3 Stunden zum Sieden erhitzt und nach Abkühlung auf Raumtemperatur aufgearbeitet.

Zur Aufarbeitung gibt man 15 g Kieselgel 60 (Merck) zu und destilliert das Lösungsmittel vorsichtig am Rotationsverdampfer ab. Das so erhaltene Adsorbat gibt man auf eine Säule auf (150 g Kieselgel, Durchmesser: 3 cm, Länge: 60 cm), die mit Aluminiumfolie gegen Lichteinfluß geschützt wird. Elution mit Benzol/Chloroform 1:1 (Fraktionsgröße: 15 ml) ergibt unumgesetztes Ausgangsmaterial (ca. 0,5 l Eluat). Nachfolgende Elution mit Benzol/Chloroform 3:7 ergibt Verbindung 9 (ca. 1,5 l) Eluat, wobei noch (4-Methyl-phenyl-sulfonyl)-hydrazid mitenthalten sein kann. Das Eluat wird am Rotationsverdampfer vorsichtig eingedampft. Die Ausbeute beträgt 5,4 g (85%) des Tosylhydrazons (verunreinigt mit (4-Methyl-phenyl-sulfonyl)-hydrazid) als Öl bzw. öliger Schaum. Aus Ethylacetat/Hexan erhält man weiße Kristalle.
Fp.: 156-159°C

3β-Acetoxy-25-hydroxy-1α,2α-epoxy-cholesta-5,7-dien

In einem 250 ml-Dreihalskolben werden 6,4 g 3β-Acetoxy-25-hydroxy-1α,2α-epoxy-cholest-5-en-7-(4-methyl-phenyl-sulfonyl)-hydrazon eingewogen und 100 ml absolutiertes Toluen (gereinigt und von Lithiumhydrid oder Lithium-aluminiumhydrid abdestilliert) zudestilliert. Der Kolben wird unter Argonbegasung mit einem Rückflußkühler versehen und mit Aluminiumfolie abgedunkelt.
Zur Steroidlösung gibt man 5 g Lithiumhydrid zu und erwärmt auf 100°C. Nach 60 Minuten ist die Umsetzung beendet. Das Reaktionsgemisch wird auf 0°C abgekühlt und langsam (Gasentwicklung!) innerhalb von 5-10 Minuten in ein Gemisch aus 200 g Eis und 100 ml Methanol gegossen ((Abzug!). Der Kolben wird mehrmals mit je 50 ml Toluen ausgespült und das Gemisch so lange gerührt, bis die Gasentwicklung beendet ist. Im Scheidetrichter trennt man die wäßrige Phase ab und wäscht die organische Phase vier- bis sechsmal mit je 100 ml destilliertem Wasser bis zur Neutralität. Anschließend wird die organische Phase über Natriumsulfat getrocknet und am Rotationsverdampfer bei 30°C eingedampft. Das verbleibende Öl (ca. 4,1 g) löst man in 20 ml Chloroform, versetzt mit 10 g Kieselgel 60 und destilliert das Chloroform vorsichtig am Rotationsverdampfer ab. Das erhaltene Adsorbat gibt man auf eine Säule auf (20g Kieselgel 60, Durchmesser: 20 mm, Länge: 80 cm) und eluiert mit Benzol/Chloroform 6:4. Die dünnschichtchromatographisch einheitlichen Fraktionen werden vereinigt und am Rotationsverdampfer eingedampft. Das zurückbleibende ölige Dien wird durch mehrfaches Abziehen mit Methanol im Vakuum in einen festen Schaum überführt, der im Ölpumpenvakuum weiter getrocknet wird (3,2 g; 70% Ausbeute, Schmelzbereich 70-90°C). Der Schaum wird gut verschlossen unter Argon im Kühlschrank aufbewahrt.
UV($CH_3OH$):$\lambda_{max}$=268nm;
$^1$H-NMR($CDCl_3$):0,64 ppm(s,3H,18-H); 1,06(s,3H,19-H); 0,97(d,3H,J 6,5Hz,21-H); 1,22(s,6H,26- und 27-H); 2,10(s,3H,$CH_3$CO); 3,06(d,1H,J 3Hz,1-H); 3,28(d,1H, J 2,5Hz,2-H); 5,40(m,1H,6- oder 7-H); 5,75(m,1H,7- oder 6-H).

3β-Acetoxy-25-hydroxy-1β,2β-epoxy-cholest-5-en-7-on

100 mg 3β-Acetoxy-25-hydroxy-cholesta-1,5-dien-7-on in 10 ml Toluol werden mit 320 mg m-Chlorperbenzoesäure versetzt und 4 Stunden bei 40°C gehalten. Die Reaktionslösung wird mit verdünnter Natriumbicarbonatlösung ausgeschüttelt, die organische Phase neutral gewaschen und getrocknet. Das nach Aodestillieren des Lösungsmittels erhaltene Rohprodukt (96 mg) wird an Kieselgel (Woelm) mit Benzol/Essigester 75:25 v/v chromatographiert.
Ausbeute: 30 mg amorphe Substanz
$[\alpha]_D^{20}$ +12° ($CH_3OH$)

2β-Acetoxy-1α-brom-3β,25-dihydroxy-cholest-5-en-7-on

In einen 100 ml Kolben werden 200 mg 3β-Acetoxy-25-hydroxy-cholesta-1,5-dien-7-on (0,438 mmol) in 40 ml THF gelöst und mit 3 bis 4 Tropfen Wasser versetzt. Zu dieser Lösung gibt man 1,05 g N-Bromacetamid (7,6 mmol) und rührt bei Raumtemperatur. Nach einer kurzen Induktionsperiode tritt eine orange Färbung auf, die im Verlauf der Reaktion in ein dunkles Rot übergeht. Im allgemeinen ist die Reaktion nach 2 bis 3 Stunden beendet. Durch Zugabe von 300 ml Eiswasser wird das Produkt ausgefällt, abgesaugt, mit Wasser gewaschen, getrocknet und aus Benzol/Aceton umkristallisiert. Man erhält 240 mg (0,4 mmol) der Titelverbindung (91,3%).
Fp.: 183-186°C
$[\alpha]_D^{20}$ (c=1, $CHCl_3$) =-22,5°

3β-Acetoxy-1β,2β-epoxy-25-hydroxy-cholest-5-en-7-on

55 mg des im vorstehenden Beispiel hergestellten Bromhydrins werden in 18 ml mit Natriumacetat bei Raumtemperatur gesättigtem absoluten Ethanol gelöst. Die Lösung wird eine Stunde bei 80°C gerührt und anschließend im Vakuum eingedampft. Das Rohprodukt wird mit Eiswasser behandelt. Der Feststoff wird abgesaugt und aus Aceton kristallisiert. Man erhält das Epoxid in weißer kristalliner Form.
Fp.: 91-92°C

5-Chlor-2-methyl-pentan-2-ol

Zu einer aus 44 g Magnesium (1,8 Mol) und 112,6 ml (1,8 Mol) Methyljodid in 450 ml Diethylether hergestellten Methylmagnesiumjodid-Lösung werden unter Rühren und Feuchtigkeitsausschluß bei - 5° bis +5°C 110 ml (0,79 Mol) 4-Chlorbuttersäureethylester in 80 ml Diethylether zugetropft. Nach 1 Std. wird mit Eis zersetzt und mit etwa 120 ml 6 n Salzsäure werden die gebildeten Salze aufgelöst und die Lösung auf einen pH-Wert von 7 eingestellt. Die organische Phase wird abgetrennt, die wässrige Phase nochmals mit Diethylether ausgeschüttelt, die vereinigten organischen Phasen mit Wasser gewaschen, dann über $Na_2SO_4$ getrocknet und der Ether abdestilliert.

Durch fraktionierte Destillation (unter Argon) werden 62,4 g (78 % d. Th) 5-Chlor-2-methyl-pentan-2-ol als schwach gelbe Flüssigkeit vom $K_{p18}$. 72-75°C erhalten. Die Aufbewahrung erfolgt vorteilhaft über $K_2CO_3$. Die Darstellung dieser Verbindung durch Umsetzung von 1-Chloro-5-hexanon mit Methylgrignardlösung ist in DE 3525801 beschrieben.

5-Chlor-2-methyl-2-trimethylsilylox-pentan

41 g 5-Chlor-2-methyl-pentan-2-ol werden in 500 ml trockenem Chloroform gelöst, 27,3 g Imidazol wird hinzugefügt und unter schwacher Kühlung und Feuchtigkeitsausschluß 49,1 ml Trimethylchlorsilan unter Rühren zugetropft. Nach 1 Std. wird das gebildete Imidazolhydrochlorid abfiltriert, die Chloroform-Lösung mit $Na_2SO_4$ und $K_2CO_3$ intensiv gerührt, dann vom Trockenmittel abfiltriert und das Chloroform unter Zugabe von Kaliumhydroxid-Splittern bei vermindertem Druck abdestilliert. Durch fraktionierte Destillation werden 38,5 g (61 %) 5-Chlor-2-methyl-trimethylsilyloxy-pentan als farblose Flüssigkeit vom $K_{p18}$ 74-76°C erhalten. Die Aufbewahrung erfolgt vorteilhaft über $K_2CO_3$.

3β,25-Dihydroxy-24-homo-cholest-1,5-dien

Unter Feuchtigkeitsausschluß und schwachem Argondruck werden 2,28 g Magnesiumspäne mit 30 ml trockenem Tetrahydrofuran überschichtet und mit 0,27 ml 1,2-Dibromethan versetzt. Nach 5 Minuten wird bei ca. 60-70°C ein Gemisch aus 12 g 5-Chlor-2-methyl-2-trimethyl-silyloxy-pentan, 1,3 ml 1,2-Dibromethan und 100 ml trockenem Tetrahydrofuran während 30 Minuten unter Rühren zugetropft und anschließend wird die graugrüne Lösung noch 2 Std. bei etwa 75°C weitergerührt. Nach dem Abkühlen wird die Grignard-Lösung mit einer Sicherheitspipette unter Argon vom unumgesetzten Magnesium abgezogen, in einen Sulfierkolben überführt und auf etwa -10°C abgekühlt. Unter Rühren und Argon als Schutzgas wird eine Lösung von 3 g (20S)-20-Tosyloxymethyl-1,5-pregnadien-3β-ol in 60 ml trockenem Tetrahydrofuran zugegeben. Unmittelbar anschließend werden 110 mg fein gepulvertes und trockenes Kupfer-I-jodid hinzugefügt und weitere 2 Std. bei 0°C gerührt und anschließend bei Raumtemperatur über Nacht stehengelassen. Dann wird das Gemisch in 500 ml gesättigter Ammoniumchlorid-Lösung aufgenommen, die organische Phase abgetrennt und die wässrige Phase mit Benzol nachextrahiert. Nach Vereinigen der organischen Phasen, Neutralwaschen, Trocknen mit $Na_2SO_4$, Filtrieren und Einengen am Vakuumrotationsverdampfer erhält man ein leicht gelbliches Öl. Zur Abspaltung der Schutzgruppe wird in 100 ml 80%igem Tetrahydrofuran gelöst und mit 1,2 g p-Toluolsulfonsäure versetzt. Nach 1 Std. Stehenlassen bei Raumtemperatur wird das Gemisch mit wässriger Natriumhydrogencarbonat-Lösung auf einen pH-Wert von 7 eingestellt und mit Chloroform extrahiert. Die organische Phase wird über $Na_2SO_4$ getrocknet, filtriert und im Vakuum eingeengt.
Nach mehrmaligem Abziehen mit trockenem Aceton erhält man 1,95 g der Titelverbindung (76 % d.Th) als farblose Kristalle, die nach Umkristallisieren aus Aceton bei 162-164°C schmelzen;
$[\alpha]_D^{20}$ (c=1,$CHCl_3$) -6°

3β-Acetoxy-24-homo-cholest-1,5-dien-25-ol

2,3 g 3β,25-Dihydroxy-24-homo-cholest-1,5-dien werden in 10 ml trockenem Pyridin gelöst, auf -5°C abgekühlt und unter Rühren mit 4 ml Essigsäureanhydrid versetzt. Danach wird bei Raumtemperatur über Nacht stehengelassen. Zur Aufarbeitung wird der Ansatz unter intensivem Durchschütteln auf Eiswasser gegossen. Dabei fallen 2,1 g 3β-Acetoxy-24-homo-cholest-1,5-dien-25-ol als farblose Kristalle aus;
Fp.: 103-104°C.

3,25-Dihydroxy-24-homo-23,24-dimethyl-cholesta-1,5-dien

4,0 g (9,65 mMol) 3β-Hydroxy-24-bishomo-chola-1,5-diensäuremethylester, gelöst in 40 ml Diethylether, werden bei 0°C langsam mit 23 ml einer 1,74molaren Etherlösung von Ethylmagnesiumbromid (40,0 mMol) versetzt. Nach 2 Std. Schütteln, wobei sich die Reaktionsmischung auf Raumtemperatur erwärmt, wird vorsichtig Eiswasser zugegeben. Danach werden 40 ml gesättigte wäßrige Ammoniumchlorid-Lösung zugesetzt. Die Etherphase wird abgetrennt und die wäßrige Lösung noch zweimal mit je 50 ml Dichlormethan extrahiert. Die vereinigten organischen Phasen werden mit Natriumsulfat und Natriumcarbonat getrocknet. Nach Abdestillieren der Lösungsmittel wird der Rückstand aus Ether/Methanol (20:1) umkristallisiert.
Ausbeute: 3,12 g (73 %), weiße Kristalle
Fp. 144-145°C, $[\alpha]_D^{20}$ (c=1,$CHCl_3$) 0°.

Die weitere Umsetzung (Oxidation in 7-Position, Epoxidierung, Hydrozidbildung, Einführung der Doppelbindung) zum letztendlich gewünschten Produkt der allgemeinen Formel Ia erfolgt analog zu den vorstehenden Synthesebeispielen.

**Herstellung der Ausgangsprodukte zur Grignardierung**

1. 3β-Hydroxy-24-bishomo-chola-1,5-dien-säure und deren Methylester

500 mg (1,13 mMol) 3β-Hydroxy-20-iodmethyl-1,5-pregnadien, gelöst in 30 ml DMF werden unter Luft- und Feuchtigkeitsausschluß mit 500 mg (1,62 mMol) wasserfreiem Mangan(II)-iodid und 340 mg (1,13 mMol) Nickelchelatkomplex der allgemeinen Formel VI bzw. VI' (L=2,2'-Dipyridyl) versetzt.

Nach 8 Std. Rühren bei 30°C ist die zunächst weinrote Suspension des Nickelkomplexes klar und grün gefärbt. Es werden nochmals 185 mg (0,61 mMol) und nach weiteren 8 Std. 145 mg (0,48 mMol) des Nickelchelats zugegeben. Die rote Farbe der Reaktionsmischung bleibt nun erhalten. Parallel zur Farbänderung wird der Verlauf der Reaktion mittels Dünnschichtchromatografie (DC) verfolgt. Das Lösungsmittel wird im Vakuum abgezogen, der Rückstand mit Wasser und verdünnter Salzsäure aufgenommen, die wässrige Phase mehrmals mit Chloroform extrahiert, die organische Phase mit Wasser, Thiosulfatlösung und nochmals mit Wasser gewaschen und über Natriumsulfat getrocknet. Das Extraktionsmittel wird vollständig abdestilliert und der Rückstand aus Methanol umkristallisiert.
Ausbeute: 330 mg, $F_p$= 180-182°C.

330 mg (0,82 mMol) der so hergestellten Steroidcarbonsäure werden in wässrigem Methanol mit etherischem Diazomethan verestert. Das Lösungsmittel wird abdestilliert und der Rückstand aus Methanol umkristallisiert.
Ausbeute: 284 mg, $[\alpha]_D^{20}$=-5°(in Chloroform)

Sowohl die Säure als auch der Methylester erweisen sich in der HPLC als jeweils einheitliche Verbindungen.

3β-Hydroxy-24-bishomo-chol-5-en-säure und deren Methylester

800 mg (1,81 mMol) 3β-Hydroxy-20-iodmethyl-5-pregnen werden mit 500 mg (1,62 mMol) Mangan(II)-iodid und 1010 mg (4,18 mMol) des Nickelkomplexes der allgemeinen Formel VI bzw. VI' (L=N,N,N',N'-Tetramethylethylendiamin) in 30 ml Dimethylformamid gelöst und 2 h im Ultraschallbad zur Reaktion gebracht. Die Aufarbeitung zur Steroidcarbonsäure erfolgt analog wie unter 1.

Die Veresterung der Säure erfolgt nach einer Vorschrift von MANDAL (Ind. J. Chem. 22B (1983), 505) mit Methanol und Trimethylchlorsilan, Umkristallisation aus Aceton.
Ausbeute: 430 mg, (Methylester) $F_P$=115-119°C

20-tert. Butyl-diphenyl-silyloxy-methyl-pregna-1,5-dien-3-on (2)

8 g (24,3 mMol) (20S)-20-Hydroxymethyl-1,5-pregnadien-3-on werden in 200 ml trockenem Pyridin gelöst, mit 8 g (120 mMol) Imidazol und 13 g (45 mMol) tert.Butyldiphenylsilylchlorid versetzt und bei 40-80°C eine Stunde gerührt. Nach beendeter Reaktion (DC-Kontrolle: Kieselgel Merck, Laufmittel Benzol/Aceton 9:1) wird in Eiswasser gegossen, das abgeschiedene gelbliche Öl abgetrennt und die wässrige Schicht mit Methylenchlorid ausgeschüttelt. Dieser Extrakt wird mit dem abgetrennten Öl vereinigt, mit Methylenchlorid verdünnt, mit Wasser gewaschen, getrocknet und im Vakuum eingedampft. Man erhält 20 g eines zähen gelblichen Öles, das durch Säulenchromatographie (Kieselgel 60 Merck, 0,063-0,2 mm, Elution mit Hexan/Essigsäureethylester bis zum Verh. 80:20) gereinigt wird. Nach dem Einengen des Eluats verbleiben 11,9 g farbloses Öl (ca. 100 %), das noch Reste des Sylilierungsreag. enthält, die nur schwierig zu entfernen sind. Der Rückstand wird ohne weitere Reinigung in die nächste Stufe eingesetzt.
IR ($CHCl_3$)$\lambda_{max}$ ($cm^{-1}$): 1668, 1580, 1496, 1426, 1378.
$^1$H-NMR (200MHZ) ($CDCl_3$) $\delta$(ppm): 0,74 (s,3H, 18-H); 1,10 (s,9H, -$C(CH_3)_3$); 1,17 und 1,19 (d, 3H, 21-H); 1,23 (s,3H, 19-H); 2,88 und 2,96 (d, 1H, H-4 eq.); 3,30-3,45 (m,2H, -$CH_2$-O-Si-); 5,43 und 5,44 (d,1H,6-H); 5,88-5,93 und 6,92-7,62 (dd, jeweils 1H, AB-System 1-H und 2-H); 7,38-7,78 (m, aromatische H).

20-tert. Butyldiphenylsilyloxymethyl-3β-hydroxy-pregna-1,5-dien (3)

6 g (150 mMol) Natriumborhydrid werden in 250 ml absol. Ethanol gelöst, die Lösung auf -25°C abgekühlt und unter Rühren innerhalb 30 Minuten mit 10 g (90 mMol) wasserfreiem Calciumchlorid in 250 ml absol. Ethanol unter Rühren versetzt. Man rührt bei -25°C weitere 30 Minuten und gibt die Mischung anschließend innerhalb von 10 Minuten zu einer auf -25°C gekühlten Lösung von 10 g (18 mMol) 2 in 350 ml absol. Ethanol.

Die Mischung wird 30 Minuten bei tiefer Temperatur gerührt und nach beendeter Umsetzung (DC-Kontrolle: Kieselgel Merck, Laufmittel Benzol/Aceton 9:1) mit 200 ml Aceton, 350 ml Wasser verdünnt und mit 6n HCl auf einen pH von 3 gebracht. Man verdünnt mit einer größeren Menge Wasser und schüttelt mit Methylenchlorid aus, wäscht die organische Phase mit gesättigter Kochsalzlösung, trocknet und engt im Vakuum ein.

Der Rückstand (11 g) wird durch Säulenchromatographie (Kieselgel 60 Merck, 0,063 - 0,2 mm, Elution mit Hexan/Essigsäureethylester bis zum Verh. 60:40) gereinigt. Man erhält 7,9 g (77 %) kristallines Produkt,
Fp. 138 - 142°C,

20-tert.Butyldiphenylsilyloxymethyl-3β-isobutyloxycarbonyloxypregna-1,5-dien (4)

4,5 g (7,5 mMol) 3 werden in 60 ml Pyridin (getrocknet über KOH) gelöst, mit 1 ml (10 mMol) Chlorkohlensäureisobutylester versetzt und auf 40°C erwärmt. Nach erfolgter Umsetzung (ca. 30 Minuten, DC-Kontrolle: Kieselgel Merck, Laufmittel Benzol/Aceton 9:1) wird die Mischung in Eiswasser gegossen und vom gebildeten Niederschlag abgesaugt. Der Niederschlag wird mit Wasser gewaschen, in Methylenchlorid aufgenommen, mehrmals mit Wasser gewaschen, die Lösung getrocknet und im Vakuum eingeengt.
Man erhält 5,1 g (97 %) einer zähen gelben Masse, die aus Methanol umkristallisiert wird.
Ausbeute: 4,8 g (90 %)
Fp. 98 - 101°C

3β-Isobutyloxycarbonyloxy-22-tert.butyl-diphenyl-silyloxy-pregna-1,5-dien-7-on (5)

3,2 g (4,7 mMol) 4 werden in 25 ml absol. Benzol gelöst und mit 0,2 g (0,8 mMol) wasserfreiem Natriumdichromat, das mit 0,2 g Kieselgel (Merck 60, 0,2-0,5 mm) verrieben wurde, versetzt. Man fügt 12 ml (20 mMol) tert.-Butylhydroperoxid (80%ig) zu und rührt bei 40-60°C intensiv 6 Stunden unter Argon.
Nach beendeter Umsetzung (DC-Kontrolle: Kieselgel Merck, Laufmittel Benzol/Aceton 20:1) verdünnt man mit Ether bis zur vollständigen Fällung des Chromatkomplexes, filtriert von diesem ab und engt im Vakuum ein.
Der zähe braune Rückstand (3,5 g) wird durch Säulenchromatographie (Kieselgel 60 Merck, 0,063-0,2 mm, Elution mit Hexan/Essigsäureethylester bis zum Verh. 90:10) gereinigt.
Man erhält 2,6 g (82 %) reines kristallines farbloses Produkt, das aus Methanol umkristallisiert werden kann;
Fp. 108-112°C.

**Patentansprüche**

1. Ausgangsverbindungen zur Herstellung von Calcitriol (1α,25-Dihydroxycholecalciferol) sowie von seitenkettenmodifizierten Abkömmlingen von Calcitriol der allgemeinen Formel Ia

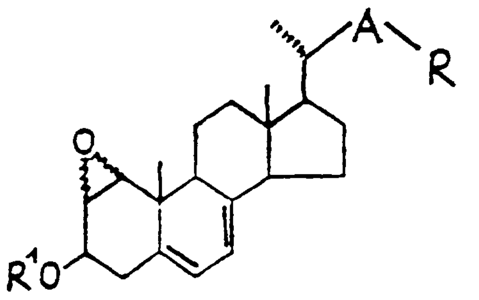

(Ia),

worin
die 1,2-Epoxygruppe α- oder β-ständig ist,
und

- $R^1$ ein Wasserstoffatom, eine gerad- oder verzweigtkettige Alkyl-, eine alkylsubstituierte Silyl-, eine Acyl-, Aroyl-, Alkoxycarbonyl- oder Alkoxyalkylgruppe jeweils mit 1 bis 6 Kohlenstoffatomen im Alkyl-, Acyl-bzw. Alkoxyrest,
- A eine Methylengruppe und
- R den Rest $-(CH_2)_n-CH_2-CR^2R^3OH$, in welchem n für eine ganze Zahl von 1 bis 7, $R^2$ und $R^3$ unabhängig voneinander jeweils für ein Wasserstoffatom, eine gerad- oder verzweigtkettige Alkylgruppe mit 1 bis 4 Kohlenstoffatomen oder $R^2$ und $R^3$ gemeinsam mit dem endständigen Kohlenstoffatom der Seitenkette für einen Cyclopropyl-, -butyl-, -pentyl-oder -hexylring stehen,

bedeuten.

**2.** Verfahren zur Herstellung von Ausgangsverbindungen der allgemeinen Formel I

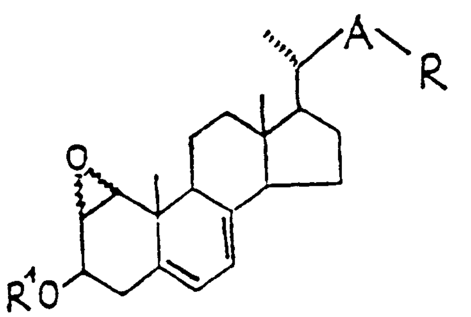

(I),

worin $R^1$, A und R die in Anspruch 1 unter der allgemeinen Formel Ia angegebene Bedeutung haben sowie A zusätzlich eine direkte Bindung und R dann einen Carbaldehydrest -CHO bedeuten, dadurch gekennzeichnet, daß (20S)-20-Hydroxymethyl-1,4-pregnadien-3-on (Formel II)

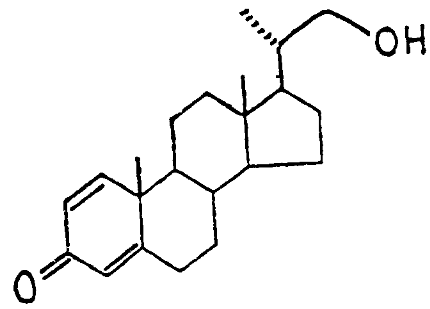

(II),

mit einem Alkalisalz eines aliphatischen $C_1$- bis $C_5$-Alkohols in etherischer Lösung zum (20S)-20-Hydroxymethyl-1,5-pregnadien-3-on (Formel III)

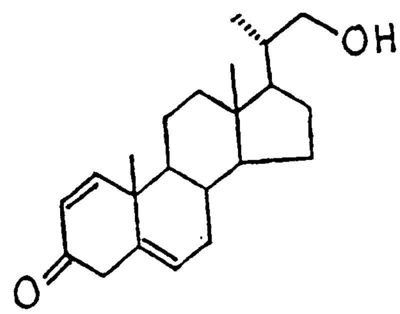

(III),

isomerisiert,

und dann entweder a), wenn in der herzustellenden Verbindung der allgemeinen Formel I A eine Methylengruppe und R den Rest, $-(CH_2)_n-CH_2-CR^2R^3OH$ bedeuten soll,
diese Verbindung der Formel III mit einem aliphatischen oder aromatischen Sulfonsäurechlorid der allgemeinen Formel $R^4-SO_2-Cl$ ($R^4$ = Phenyl- oder $C_1$- bis $C_4$-Alkylrest) unter Zusatz einer Base unterhalb oder bei Raumtemperatur in die entsprechende 20-Sulfonyloxymethyl-Verbindung der allgemeinen Formel IVa

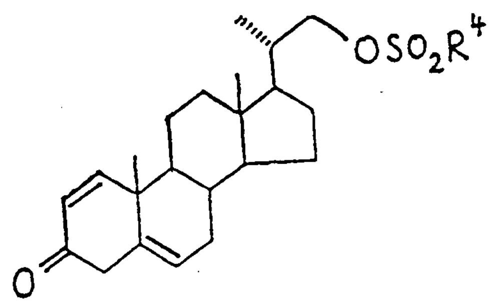

(IVa)

überführt
und diese Verbindung mit einem komplexen Metallhydrid in alkoholischer und/oder etherischer Lösung zur entsprechenden 3β-Hydroxyverbindung der Formel Va

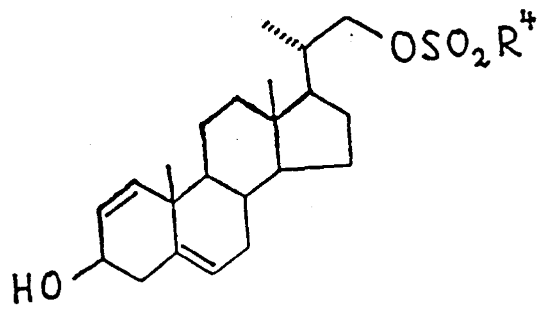

(Va)

reduziert, dann in dieser Verbindung, gegebenenfalls nach Überführung des Tosylats in das entsprechende Bromid oder Jodid, die Seitenkette entweder durch bekannte Verfahren oder durch Umsetzung mit einem Nickelchelatkomplex der allgemeinen Formel VI

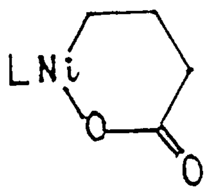

(VI)

worin
L einen 2,2'-Dipyridylrest (dipy) oder dessen Alkylsubstitutionsprodukt oder Ethylendiamin bzw. dessen N-alkylierte Derivate bedeutet, in organischen Lösungsmitteln oder Lösungsmittelgemischen bei einer Reaktionstemperatur zwischen Raumtemperatur und 55°C und unter Zusatz eines wasserfreien Mangan(II)-salzes, wobei für L=dipy die Reaktion unter heterogenen Bedingungen und für L=Ethylendiamin bzw. dessen N-alkylierte Derivate in homogener Phase bei gleichzeitiger Einwirkung von Ultraschall durchgeführt wird und die Reaktionsprodukte sauer hydrolysiert werden, unter Erhalt einer Verbindung der allgemeinen Formel VIIa

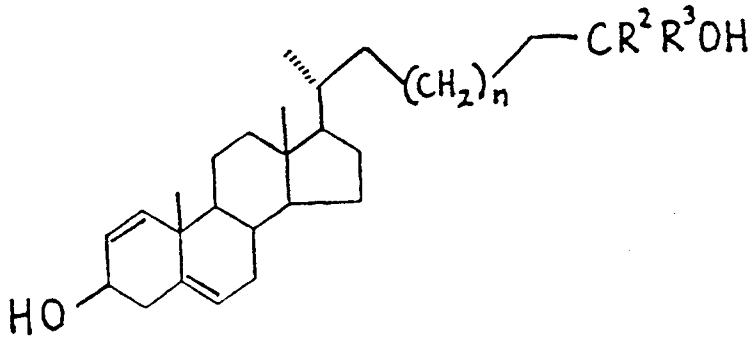

(VIIa)

worin $R^2$ und $R^3$ die bereits angegebene Bedeutung haben, bzw. im Falle, daß mit VI umgesetzt wurde, eine Verbindung der allgemeinen Formel VIIa'

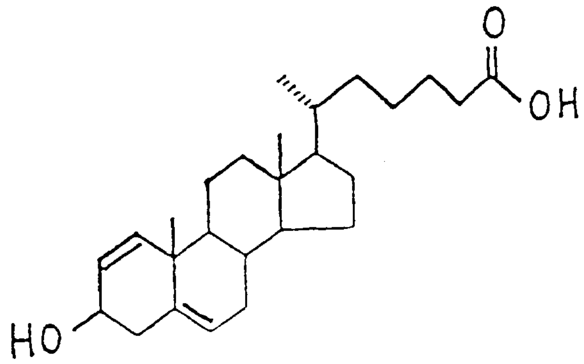

(VIIa')

deren Säuregruppe gegebenenfalls derivatisiert werden kann, aufgebaut, und anschließend eine Verbindung der allgemeinen Formel VIIa oder VIIa' im Fall der Verbindung der allgemeinen Formel VIIa' nach deren doppelten Grignardierung mit einer Verbindung $R^{23}MgHal$ ($R^{23}=R^2=R^3$; Hal=Cl, Br, J), zu einer Verbindung der allgemeinen Formel VIIIa

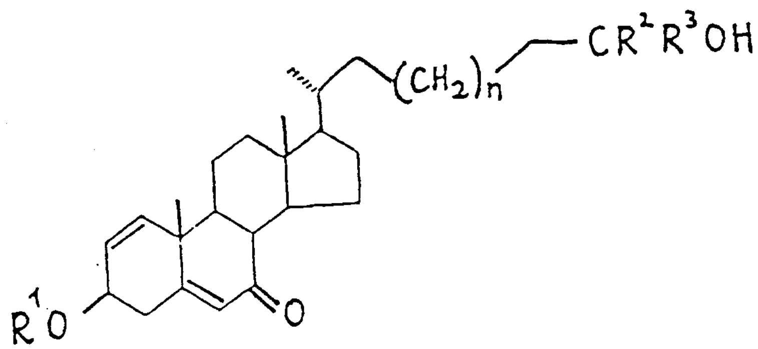

(VIIIa)

nach Schutz der sekundären OH-Gruppe, oxidiert und diese gegebenenfalls, wenn nicht $R^1$ bereits eine alkylsubstituierte Silylgruppe bedeutet, über die freie 3-Hydroxyverbindung in den entsprechenden 3-Silylether überführt und dieser zu einer Verbindung der allgemeinen Formel IXa

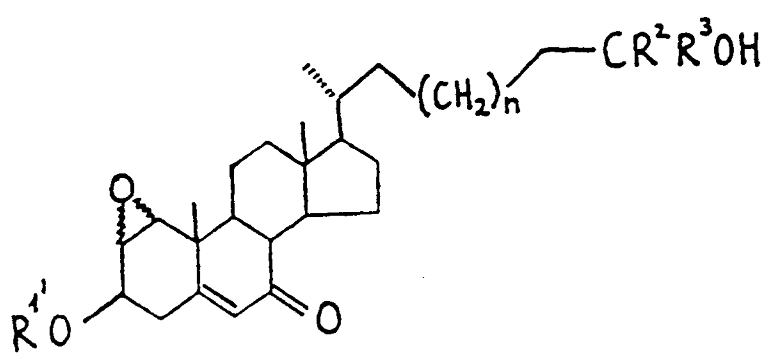

(IXa)

worin $R^{1'}$ den alkylsubstituierten Silylrest bedeutet, (1,2α-Epoxid) oder, wenn nicht $R^1$ bereits eine Acyl- oder Aroylgruppe bedeutet, über die freie 3-Hydroxyverbindung in die entsprechende 3-Acyloxyverbindung überführt, und diese zu einer Verbindung der allgemeinen Formel IXa, worin $R^{1'}$ eine Acyl-oder Aroylgruppe bedeutet (1,2β-Epoxid) epoxidiert, oder die Verbindung der allgemeinen Formel VIIIa, worin $R^1$ eine Acyl- oder Aroylgruppe bedeutet, mit einer positiviertes Halogen, insbesondere positiviertes Brom enthaltenden Verbindung in eine Verbindung der allgemeinen Formel XI

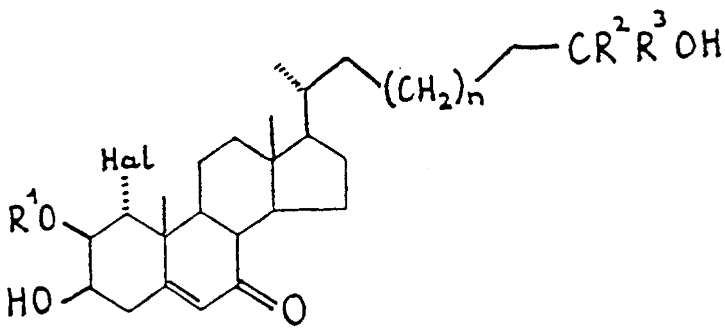

(XI)

überführt und diese mit einer schwachen Base in ein 1,2β-Epoxid der allgemeinen Formel IXaβ

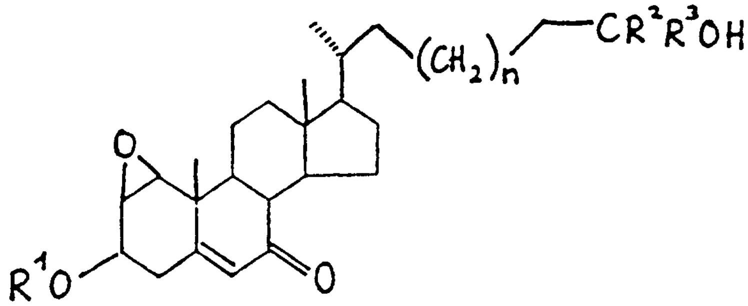

(IXaβ)

umgewandelt, gegebenenfalls in IXa der 3-Silylether gespalten und über die freie 3-Hydroxyverbindung gegebenenfalls in die entsprechende 3-Acyloxyverbindung (IXa mit $R^{1'}$ = Acyl) umgewandelt und mit Tosylhydrazin $CH_3C_6H_4$-$SO_2$-NH-$NH_2$ in das Tosylhydrazid der allgemeinen Formel Xa

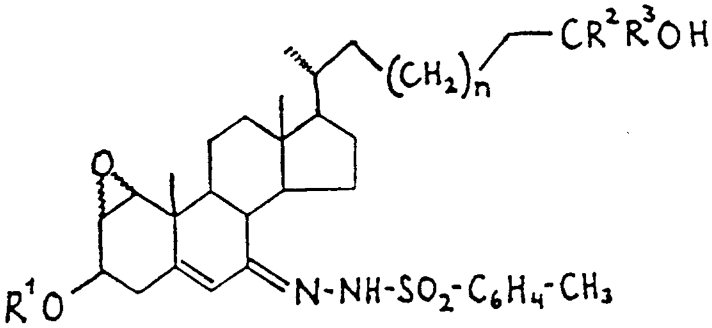

(Xa)

überführt und dieses mit Lithiumhydrid zu einer Verbindung der allgemeinen Formel Ia

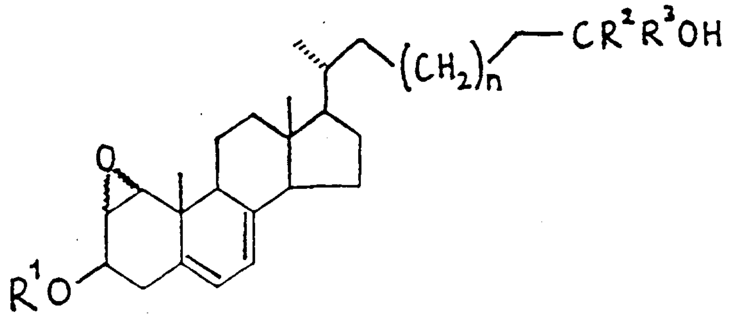

(Ia)

umgesetzt

oder b), wenn in der herzustellenden Verbindung der allgemeinen Formel I A eine direkte Bindung und R einen Carbaldehydrest -CHO bedeuten soll, in der Verbindung der Formel III eine leicht abspaltbare Schutzgruppe $R^X$ unter Erhalt des entsprechenden (20S)-20-substituierten-Methyl-1,5-pregnadien-3on IVb

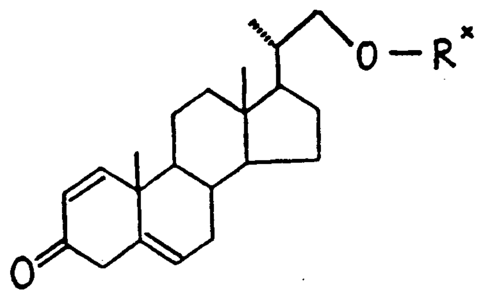

(IVb)

eingeführt,

und dieses mit einem komplexen Metallhydrid analog dem Reaktionsschritt IVa→Va zur entsprechenden 3-Hydroxyverbindung der allgemeinen Formel Vb

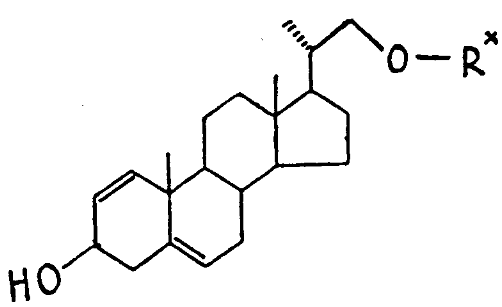

(Vb)

reduziert und darin die 3-Hydroxygruppe mit einem Alkoxycarbonylchlorid der allgemeinen Formel R"OC(O)Cl (R" ist ein gerad-oder verzweigtkettiger Alkylrest mit 1 bis 6 Kohlenstoffatomen) in Gegenwart einer Base verestert und anschließend der erhaltene Ester analog dem Reaktionsschritt VIIa bzw. VIIa'→VIIIa in Allylposition zur entsprechenden Verbindung der allgemeinen Formel VIIIb

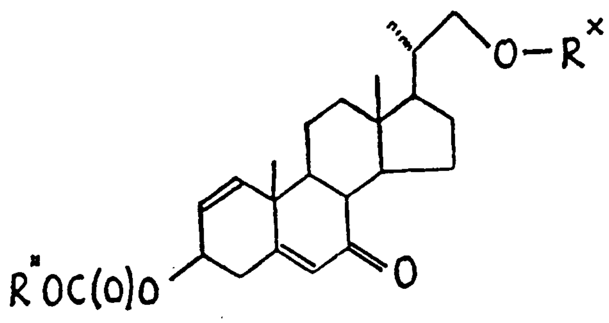

(VIIIb)

oxidiert,
diese Verbindung analog dem Reaktionsschritt VIIIa→IXa zur entsprechenden Verbindung der allgemeinen Formel IXb

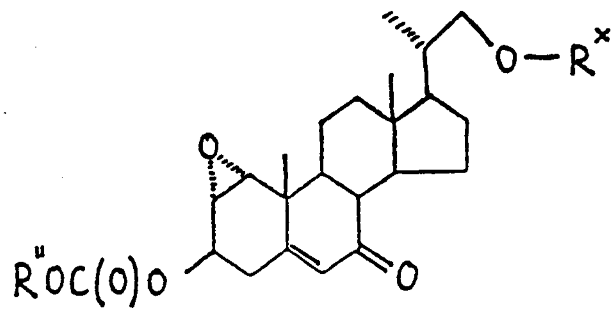

(IXb)

epoxidiert,
diese Verbindung analog dem Reaktionsschritt IXa→Xa mit Tosylhydrazon in das entsprechende Tosylhydrazid der allgemeinen Formel Xb

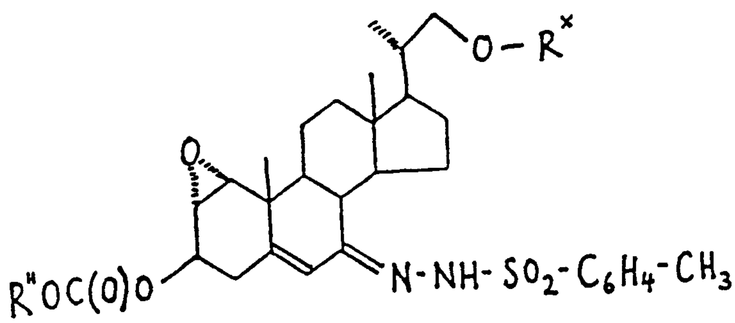

(Xb)

überführt, dieses anschließend analog dem Reaktionsschritt Xa→Ia mit Lithiumhydrid zu einer Verbindung der allgemeinen Formel Ib'

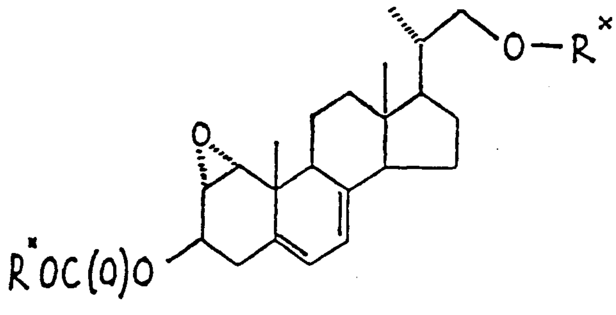

(Ib')

umgesetzt,
die Schutzgruppe $R^x$ unter milden Bedingungen abgespalten, die gebildete 20-Hydroxymethylverbindung der allgemeinen Formel Ib'' schließlich zum entsprechenden 20-Aldehyd der allgemeinen Formel Ib

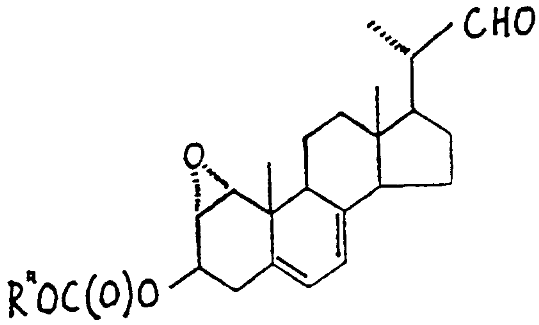

(Ib)

oxidiert und anschließend gewünschtenfalls der 3-Alkoxycarbonylester durch gängige Verfahren in einen anderen, vorstehend unter $R^1$ genannten Substituenten umgewandelt wird.

**3.** Zwischenprodukte der allgemeinen Formel VIIIa' und VIIb

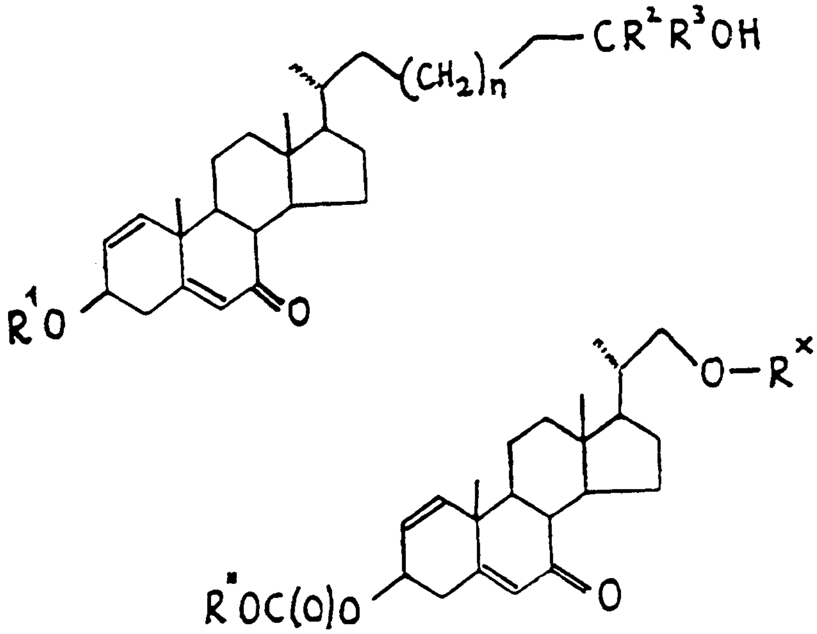

(VIIIa')

(VIIIb)

worin
$R^1$, $R^2$, $R^3$ und n bzw. R'' und $R^x$ die bereits in Anspruch 2 angegebene Bedeutung haben, wobei aber in der allgemeinen Formel VIIIa', wenn n = 1 ist und $R^2$ und $R^3$ je für eine methylgruppe stehen, $R^1$ nicht Wasserstoff oder Adamantoyl sein kann.

**4.** Zwischenprodukte der allgemeinen Formeln IXa und IXb

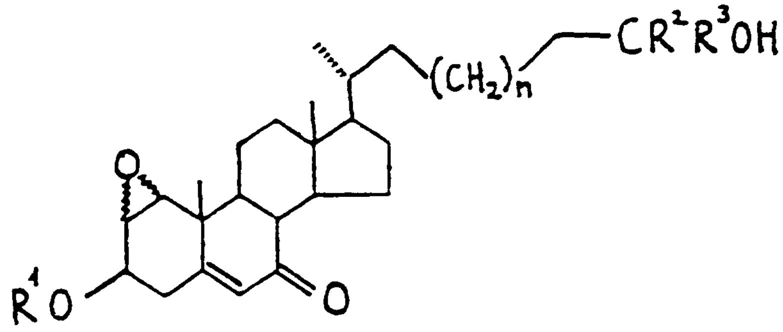

(IXa)

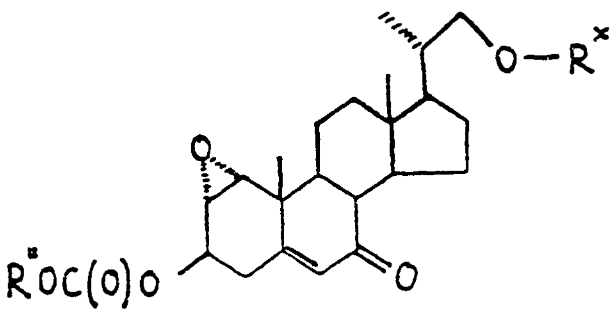

(IXb)

worin
$R^1$, $R^2$, $R^3$ und n bzw. R" und $R^x$ die bereits in Anspruch 2 angegebene Bedeutung haben.

**5.** Zwischenprodukte der allgemeinen Formeln Xa und Xb

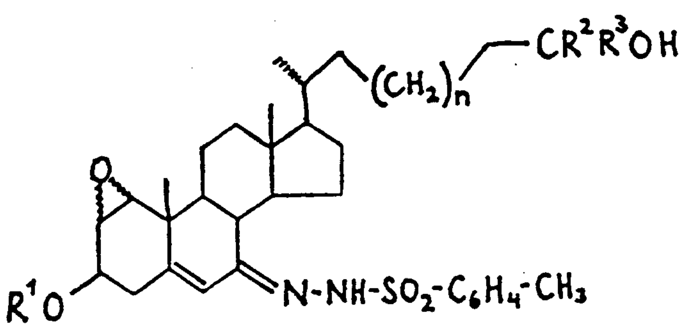

(Xa)

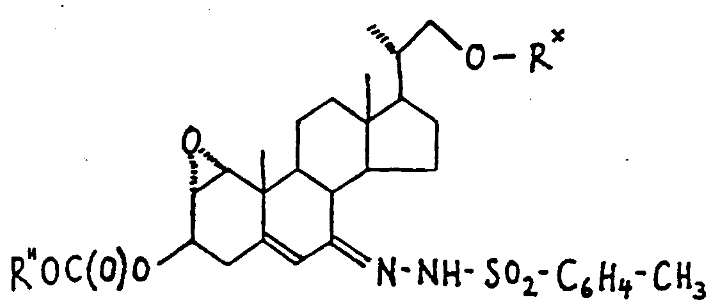

(Xb)

worin
$R^1$, $R^2$, $R^3$ und R" und $R^x$ die bereits angegebene Bedeutung haben.

## Claims

1. Starting compounds for making calcitriol (1α,25-dihydroxychole calciferol), as well as derivatives of calcitriol with modified side chains, of the general formula Ia

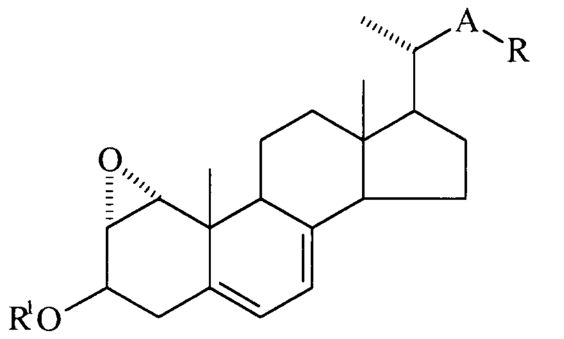

(Ia),

where
the 1,2-epoxy group is in the α- or β-position
and

R[1] represents a hydrogen atom, a straight-chain or branched-chain alkyl group, an alkyl-substituted silyl group, an acyl group, aroyl group, alkoxycarbonyl group or alkoxyalkyl group having 1 to 6 carbon atoms in the alkyl, acyl or alkoxy group,
A represents a methylene group and
R represents the group $-(CH_2)_n-CH_2-CR^2R^3OH$, where n designates a whole number from 1 to 7, $R^2$ and $R^3$ separately represent in each case a hydrogen atom, a straight-chain or branched-chain alkyl group with 1 to 4 carbon atoms or $R^2$ and $R^3$ together with the terminal carbon atom of the side chain represent a cyclopropyl, cyclobutyl, cyclopentyl or cyclohexyl ring.

2. Method for making starting compounds of the general formula I

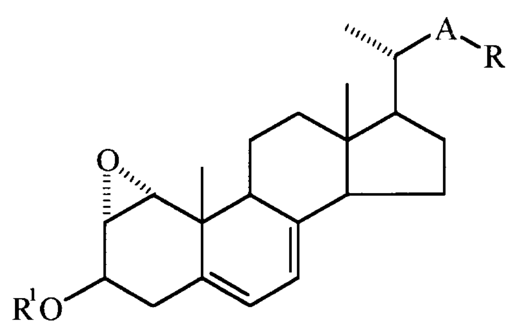

(I),

where $R^1$, A and R have the meaning indicated under the generalformula Ia in claim 1 and A additionally represents a direct bond and R then represents a carbaldehyde group -CHO, characterized in that (20S)-20-hydroxymethyl-1,4-pregnadien-3-one (formula II)

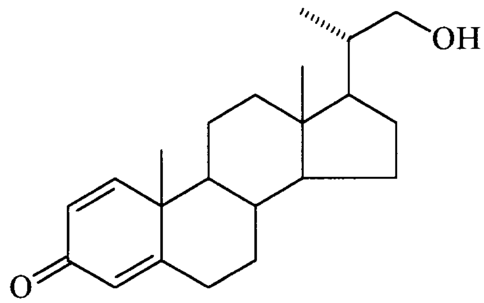

(II),

is isomerized with an alkali salt of an aliphatic $C_1$- to $C_5$-alcohol in ethereal solution to form (20S)-20-hydroxymethyl-1,5-pregnadien-3-one (formula III)

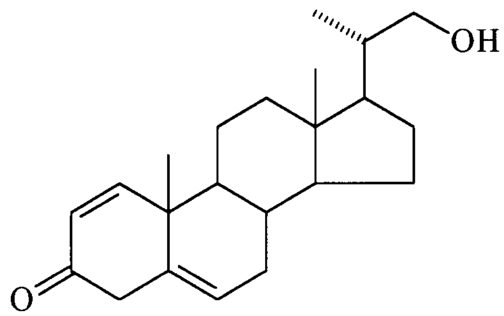

(III),

and then either

a) when A designates a methylene group and R designates the group $-(CH_2)_n-CH_2-CR^2R^3OH$ in the compound of the general formula I which is to be produced,
this compound of formula III with an aliphatic or aromatic sulfonic acid chloride of the general formula $R^4-SO_2-Cl$ ($R^4$ = phenyl- or $C_1$- to $C_4$-alkyl group) with the addition of a base at or below room temperature to form the corresponding 20-sulfonyloxymethyl compound of the general formula IVa

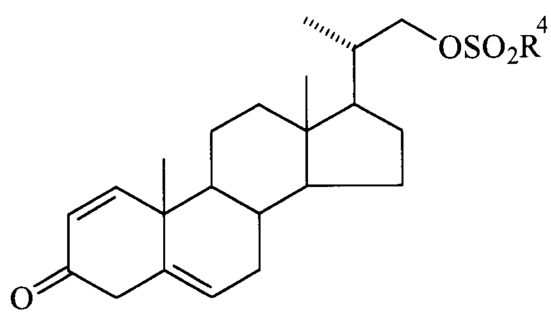

(IVa),

and this compound is reduced with a complex metal hydride in alcoholic and/or ethereal solution to form the corresponding 3β-hydroxy compound of the formula Va

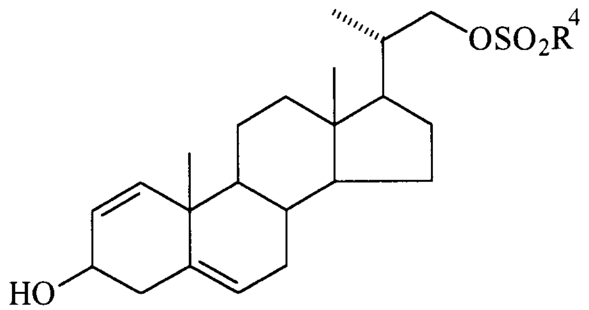

(Va)

then, possibly after converting the tosylate into the corresponding bromide or iodide, the side chain in this compound is structured either by known methods or by conversion with a nickel chelate complex of the general formula VI

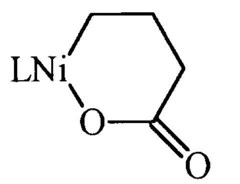

(VI)

where

L represents a 2,2'-dipyridyl group (dipy) or its alkyl-substitution product or ethylenediamine or its N-alkylated derivatives, in organic solvents or solvent mixtures at a reaction temperature between room temperature and 55°C and with the addition of an anhydrous manganese(II) salt, where for L=dipy the reaction is carried out under heterogeneous conditions and for L=ethylenediamine or its N-alkylated derivatives in homogeneous phase with simultaneous application of ultrasonics, and the reaction products are subjected to acid hydrolysis, obtaining a compound of the general formula VIIa

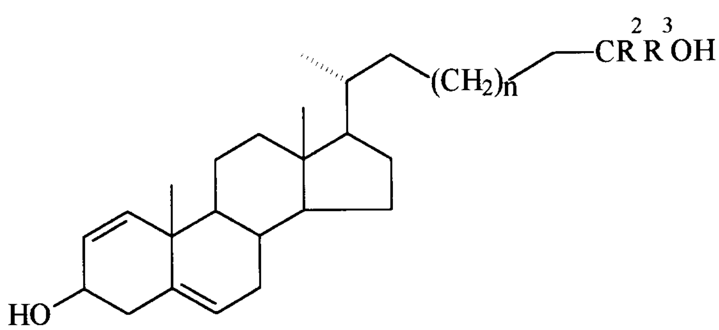

(VIIa)

where $R^2$ and $R^3$ have the same meaning as indicated above or, in the event that conversion was effected with VI, a compound of the general formula VIIa'

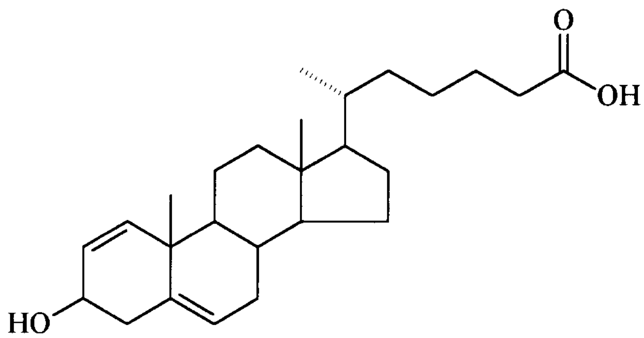

(VIIa')

whose acid group can possibly be derived, and subsequently a compound of the general formula VIIa or VIIa', in the case of the compound of the general formula VIIa' after a double Grignard reaction with a compound $R^{23}MgHal$ ($R^{23}=R^2=R^3$; Hal=Cl, Br, I), is oxidized to form a compound of the general formula VIIIa

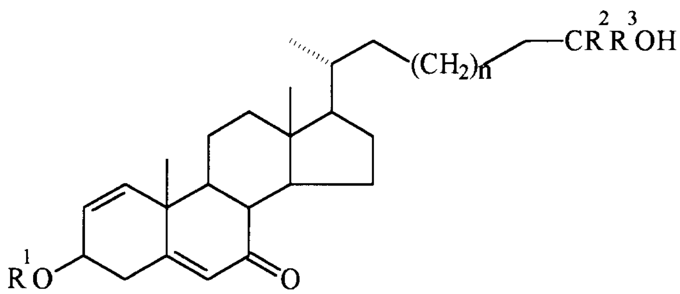

(VIIIa)

after protection of the secondary OH group, and this compound is possibly converted into the corresponding 3-silyl ether via the free 3-hydroxy compound when $R^1$ does not already designate an alkyl-substituted silyl group, and this compound is converted to a compound of the general formula IXa

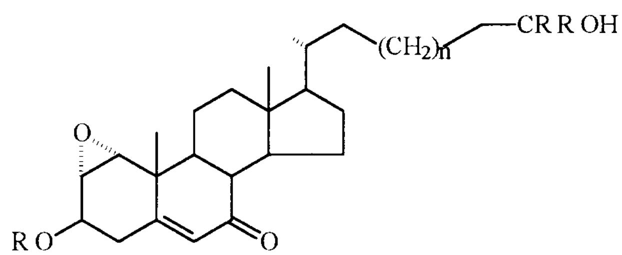

(IXa)

where $R^1$ designates the alkyl-substituted silyl group (1,2α-epoxide) or, when $R^1$ does not already designate an acyl group or aroyl group, via the free 3-hydroxy compound into the corresponding 3-acyloxy compound, and this compound is epoxidized to form a compound of the general formula IXa, where $R^1$ designates an acyl group or aroyl group (1,2β-epoxide), or the compound of the general formula VIIIa, where $R^1$ designates an acyl group or aroyl group, is converted with a compound containing a positivized halogen, particularly a positivized bromine, into a compound of the general formula XI

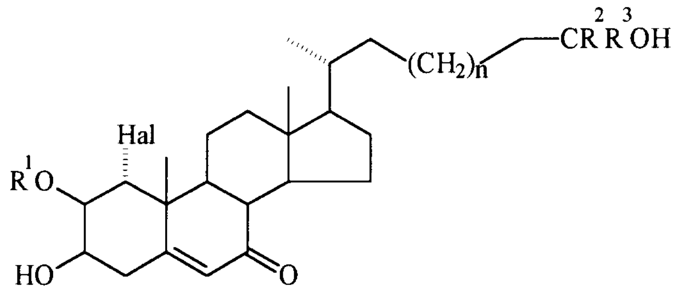

(XI)

and this is transformed by a weak base into a 1,2β-epoxide of the general formula IXaβ

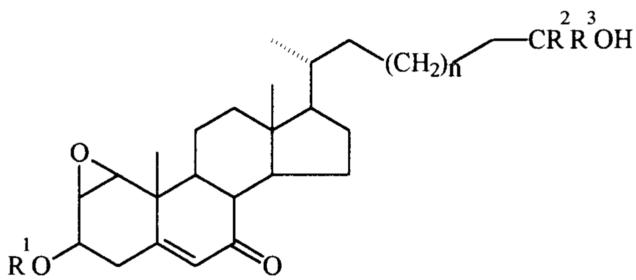

(XIaß)

the 3-silyl ether possibly being split in IXa and is possibly transformed via the free 3-hydroxy compound into the corresponding 3-acyloxy compound (IXa with $R^{1'}$ = acyl) and converted with tosylhydrazine $CH_3C_6H_4$-$SO_2$-NH-$NH_2$ into the tosylhydrazide of the general formula Xa

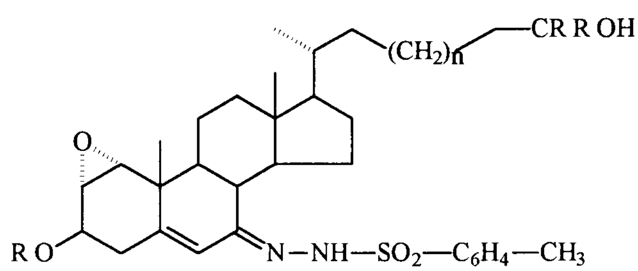

(Xa)

and this compound is converted with lithium hydride to form a compound of the general formula Ia

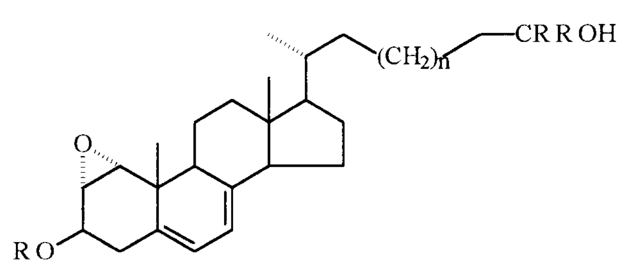

(Ia)

or

b), when A designates a direct bond and R designates a carbaldehyde group -CHO in the compound of the general formula I which is to be produced, an easily fissionable protective group $R^X$ is introduced in the compound of formula III to obtain the corresponding (20S)-20-substituted methyl-1,5-pregnadien-3-one IVb

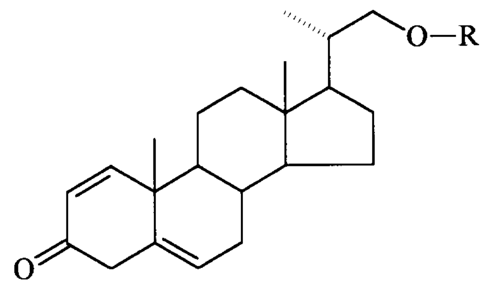

and this compound is reduced with a complex metal hydride analogous to the reaction step IVa→Va to form the corresponding 3-hydroxy compound of the general formula Vb

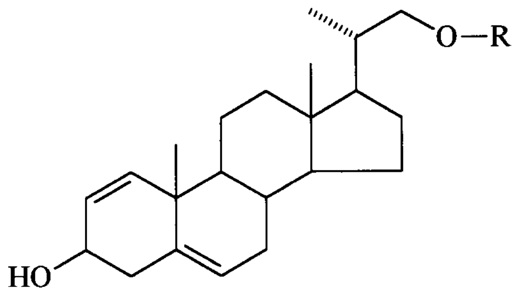

and the 3-hydroxyl group contained therein is esterized with an alkoxycarbonyl chloride of the general formula R"OC(O)Cl (R" is a straight-chain or branched-chain alkyl group with 1 to 6 carbon atoms) in the presence of a base and the obtained ester is then oxidized in a manner analogous to reaction step VIIa or VIIa'→ VIIIa in the allylic position to form the corresponding compound of the general formula VIIIb

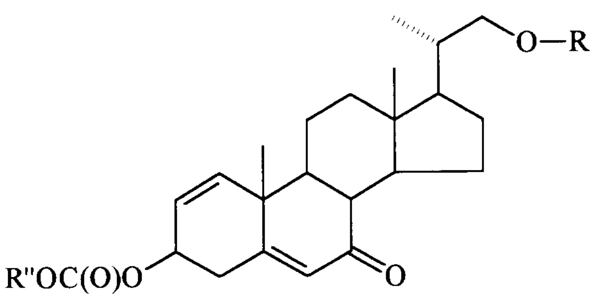

this compound is epoxidized in a manner analogous to the reaction step VIIIa→IXa to form the corresponding compound of the general formula IXb

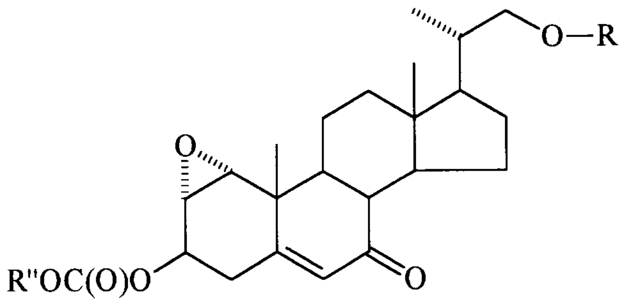

this compound is converted in a manner analogous to the reaction step IXa→Xa with tosylhydrazone to form the corresponding tosylhydrazide of the general formula Xb

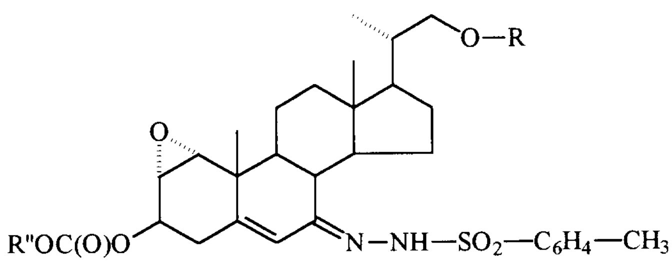

this is then converted with lithium hydride in a manner analogous to the reaction step Xa→Ia to form a compound of the general formula Ib'

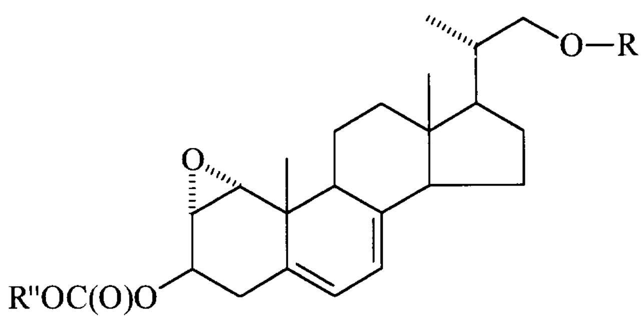

the protective group $R^X$ is split under gentle conditions, finally the formed 20-hydroxymethyl compound of the general formula Ib" is oxidized to form the corresponding 20-aldehyde of the general formula Ib

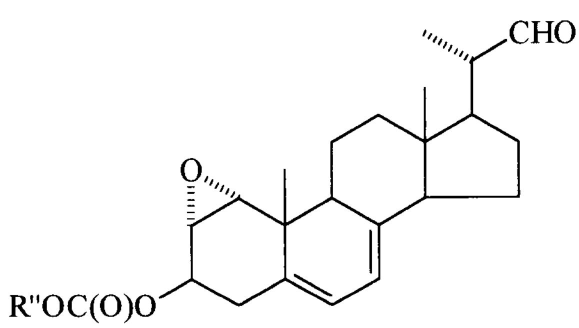

and if desired the 3-alkoxycarbonyl ester is then converted by known methods into other substituents mentioned above under $R^1$.

**3.** Intermediates of the general formulas VIIIa' and VIIIb

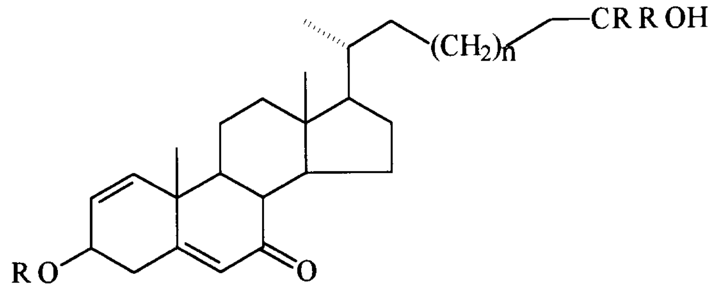

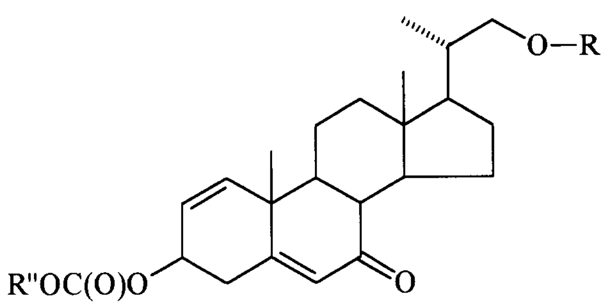

where
$R^1$, $R^2$, $R^3$ and n or R" and $R^x$ have the same meaning as already indicated in claim 2, where, however, in the general formula VIIIa', when n=1 and $R^2$ and $R^3$ each designate a methyl group, $R^1$ cannot be hydrogen or adamantoyl.

4. Intermediates of the general formulas IXa and IXb

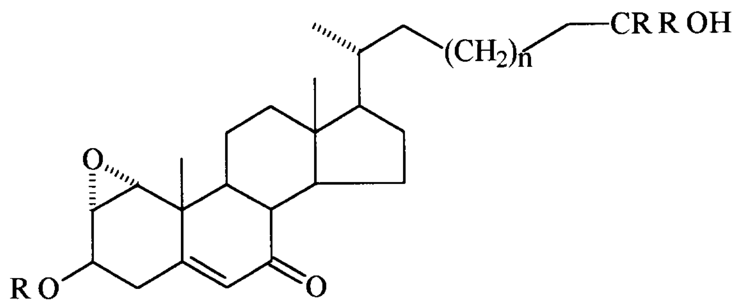

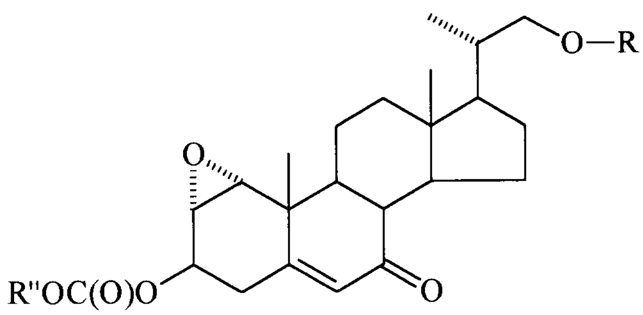

where

$R^1$, $R^2$, $R^3$ and n or R" and $R^x$ have the same meaning as already indicated in claim 2.

5. Intermediates of the general formulas Xa and Xb

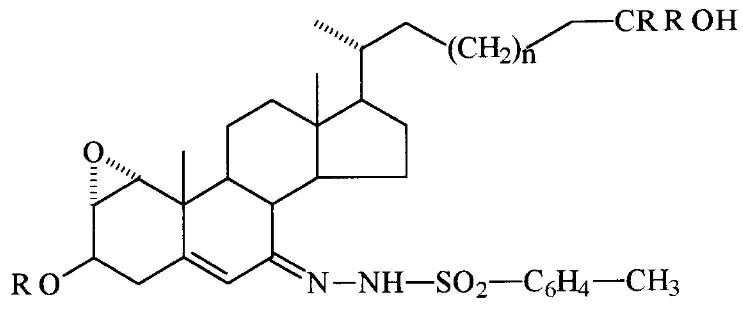

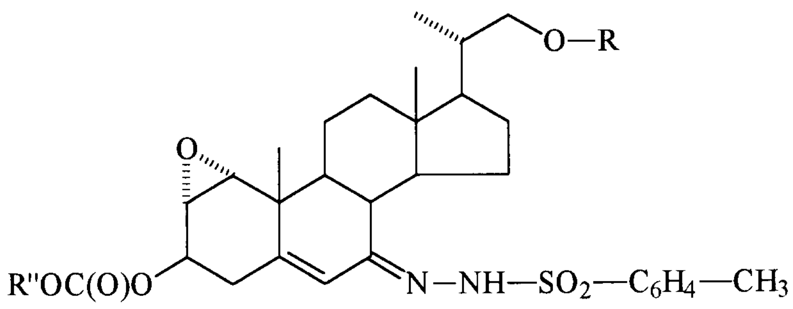

where
$R^1$, $R^2$, $R^3$ and R" and $R^x$ have the same meaning as already indicated.

## Revendications

1. Composés de départ pour la fabrication de calcitriol (1α, 25-dihydroxycholecalciférol) ainsi que des dérivés de chaîne latérale modifiée de calcitriol de la formule générale la

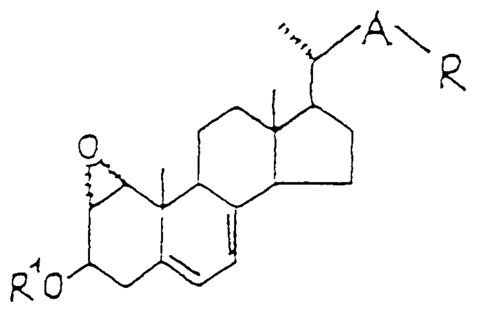

(Ia),

dans laquelle
le groupe 1,2-époxy est constant α ou β,
et

$R^1$ signifie un atome d'hydrogène, un groupe rectiligne ou à chaîne ramifiée, un groupe silyle à substitution d'alkyle, un groupe acyle, un groupe aroyle, un groupe alkoxycarbonyle ou un groupe alkoxyalkyle présentant respectivement 1 à 6 atomes d'azote dans le résidu d'alkyle, d'acyle respectivement d'alkoxy,

A signifie un groupe de méthylène et

R le résidu $-(CH_2)_n-CH_2-CR^2R^3OH$, dans lequel n représente un grand nombre de 1 à 7, $R^2$ et $R^3$ représentent indépendamnent l'un de l'autre un atome d'hydrogène, un groupe alkyle rectiligne ou à chaîne ramifiée avec 1 à 4 atomes d'azote ou $R^2$ et $R^3$ représentent, en commun avec l'atome d'azote en fin de chaîne de la chaîne latérale, un anneau cyclopropyl-,-butyl-, -pentyl- ou - hexyl.

**2.** Procédé pour la fabrication de composés de départ de la formule générale I

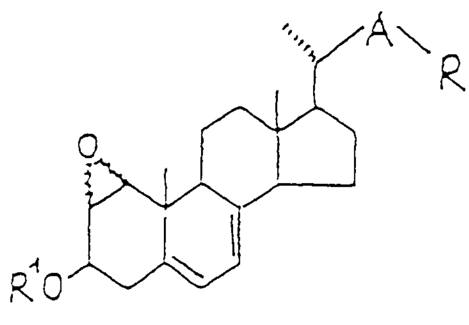

(I),

dans laquelle $R^1$, A et R ont la signfication indiquée dans la revendication 1 sous la formule générale Ia, ainsi que A signifie en plus une liaison directe et R signifie alors un résidu carbaldehyd -CHO, caractérisé en ce que (20S)-20-hydroxyméthyl-1,4-prégnadien-3-on (formule II)

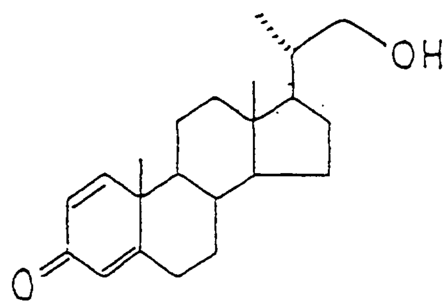

(II),

est isomérisé avec un sel alcalin d'un alcool $C_1$ à $C_5$ aliphatique dans une solution éthérée en (20S)-20-hydroxyméthyl-1,5-prégnadien-3-on (formule III)

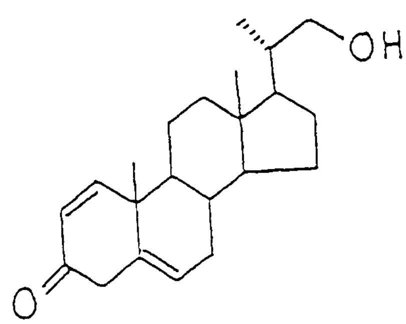

(III),

et ensuite soit a), quand, dans le composé à fabriquer de la formule générale I, A doit signifier un groupe méthylène et R le résidu $-(CH_2)_n-CH_2-CR^2R^3OH$,
ce composé de la formule III est transformé avec un chlorure d'acide sulfonique aromatique ou aliphatique de

la formule générale $R^4$-$SO_2$-Cl ($R^4$ = résidu phényl- ou $C_1$ à $C_4$-alkyle) en ajoutant une base en dessous de ou à la température ambiante en composé correspondant 20-Sulfonyloxymethyl de la formule générale IVa

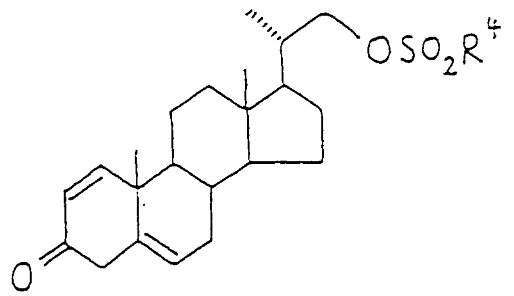

(IVa)

et ce composé est réduit avec un hydrure métallique complexe dans une solution alcoolisée et/ou éthérée au composé correspondant 3β-hydroxy de la formule Va

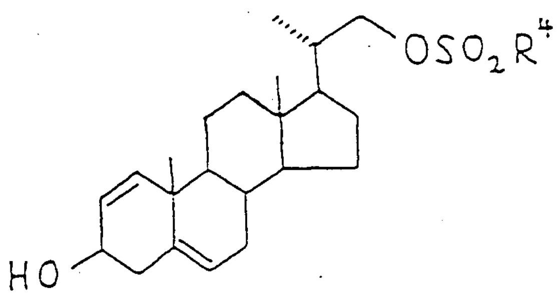

(Va)

ensuite dans ce composé éventuellement après transformation du tosylate en bromure ou iodure correspondante, la chaîne latérale est constituée soit par des procédés connus, ou soit par transformation avec un complexe de chelate de nickel de la formule générale VI

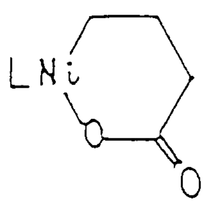

(VI)

dans laquelle
L signifie un résidu 2,2'-dipyridyle (dipy) ou son produit de substitution alkyle ou de l'éthylène diamine respectivement son dérivé N-alkylé, dans des solutions organiques ou des mélanges de solution à une température de réaction entre la température ambiante et 55°C et en ajoutant un sel anhydre de manganèse (II), pour L = dipy, la réaction est effectuée sous conditions hétérogènes et pour L = éthylènediamine respectivement son dérivé N-alkyle, est effectuée en phase homogène sous l'action simultanée d'ultrasons et les produits de réaction sont acidolysés en obtenant un composé de la formule générale VIIa

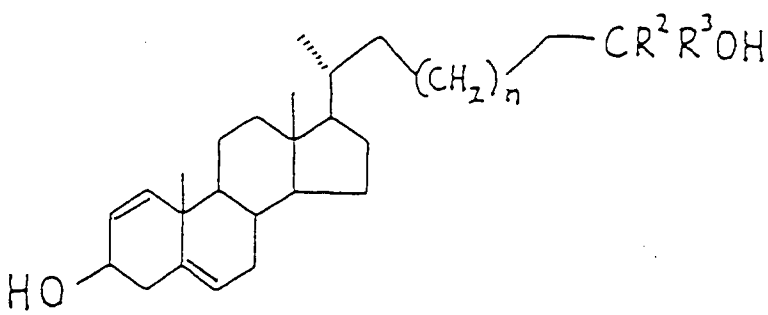

(VIIa)

dans laquelle, $R^2$ et $R^3$ ont la signification déjà indiquée, respectivement dans le cas où la transformation se produit avec VI, un composé de la formule générale VIIa' est développé

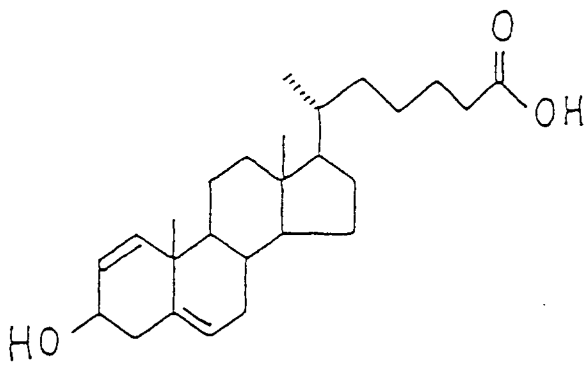

(VIIa')

dont le groupe d'acide peut éventuellement être dérivé, et ensuite un composé de la formule générale VIIa ou VIIa' est oxydé dans le cas d'un composé de la formule générale VIIa' après avoir grignardée doublement cette dernière, avec un composé $R^{23}MgHal$ ($R^{23}=R^2=R^3$; Hal=Cl, Br, J), en un composé de la formule générale VIIIa

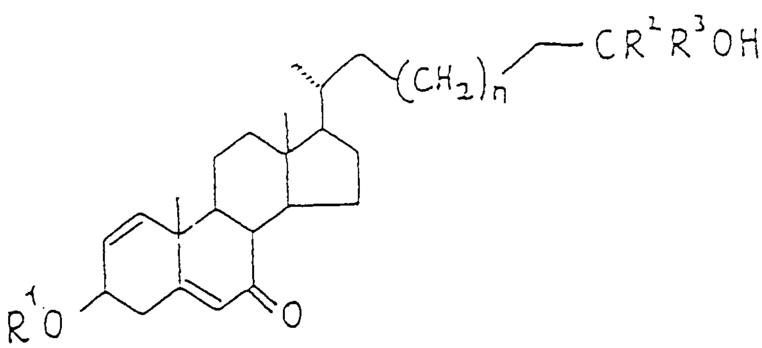

(VIIIa)

après la protection du groupe OH secondaire et ce composé est éventuellement transformé, quand $R^1$ ne signifie pas déjà un groupe silylique à substitution alkyle, par l'intermédiaire du composé 3-hydroxy libre, en 3-silyléther correspondant, et ce dernier est transformé en un composé de la formule générale IXa,

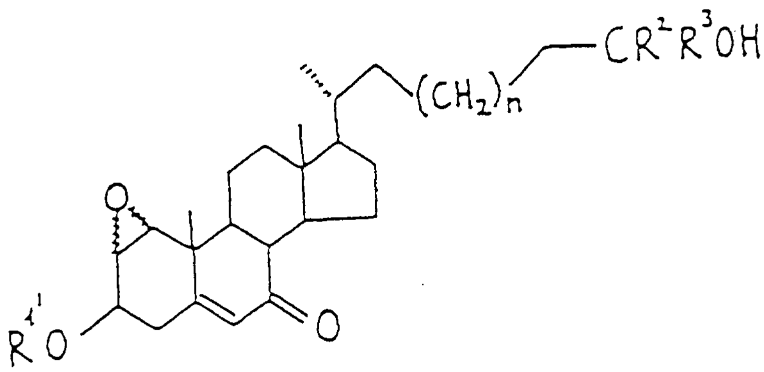

(IXa)

dans laquelle $R^1$ signifie le résidu silyle à substitution alkyke, (1,2α-époxy), ou bien, si $R^1$ ne signifie pas déjà un groupe alkyle ou aroyle, il est transformé par l'intermédiaire du composé 3-hydroxy libre, en composé 3-acyloxy correspondant et celui-ci est époxydé en un composé de la formule générale IXa, dans laquelle $R^1$ signifie un groupe acyle ou aroyle (1,2β-époxy), ou bien le composé de la formule générale VIIIa, dans laquelle $R^1$ signifie un groupe alcyle ou aroyle, est transformé, avec un halogène positivé, en particulier un composé contenant du bromure positivé, en un composé de la formule générale XI

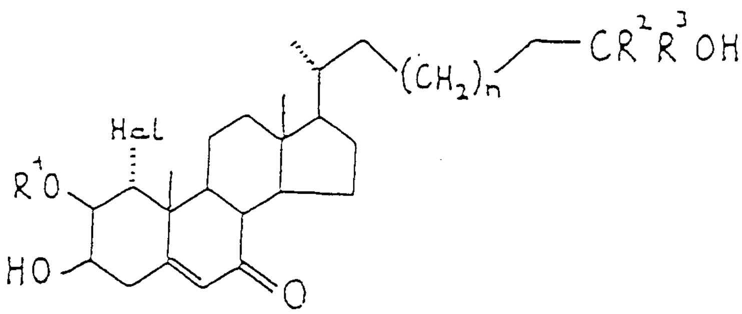

(XI)

et celui-ci est transformé, par l'intermédiaire d'une base faible, en 1,2β-époxy de la formule générale IXaβ

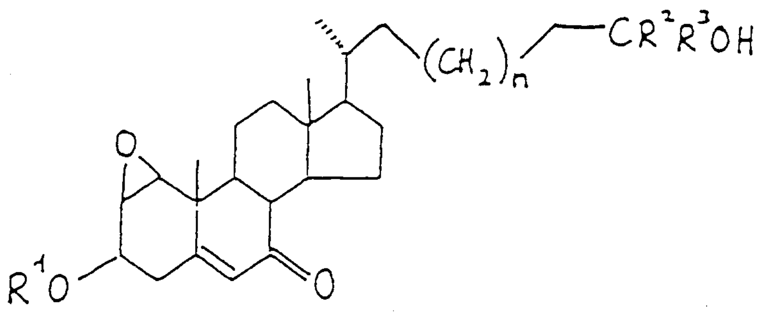

(IXaβ)

éventuellement est scindée en IXa du 3-silyléther et est transformée, par l'intermédiaire du composé libre 3-hydroxy éventuellement en composé correspondant 3-acyloxy (IXa avec $R^1$ = acyle) et est transformée, avec tosylhydrazin $CH_3C_6H_4$-$SO_2$-NH-$NH_2$ en tosylhydrazid de la formule générale Xa

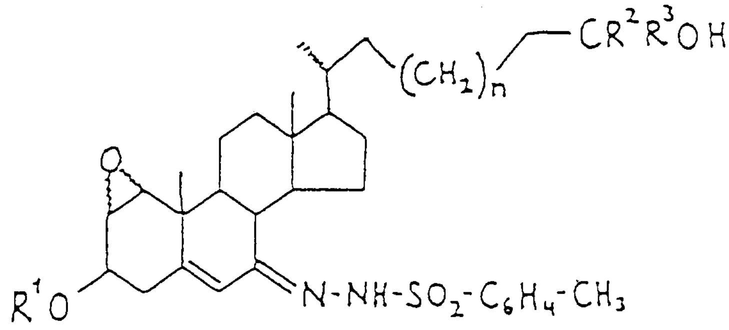

(Xa)

et ceci est transformé, avec de l'hydrure de lithium en un composé de la formule générale la

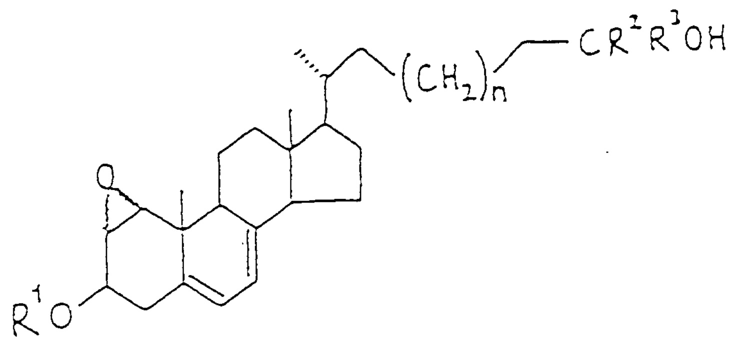

(Ia)

ou b), quand, dans le composé à produire de la formule générale I, A doit signifier une liaison directe et R un résidu carbaldehyd - CHO, dans le composé de la formule III est inséré un groupe de protection facilement séparable $R^X$ en conservant de manière correspondante (20S)-20-substitution de méthyl-1,5-prégnadien-3on IVb,

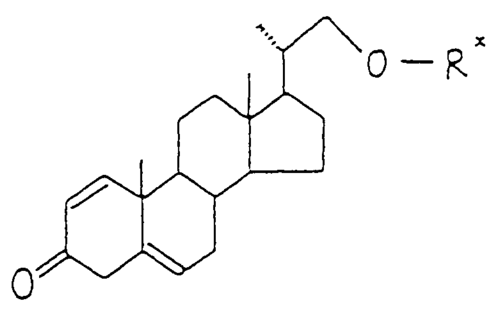

(IVb)

et ceci est réduit, avec un hydrure de métal complexe, de manière analogue à l'étape de réaction IVa→VA en un composé correspondant 3-hydroxy de la formule générale Vb

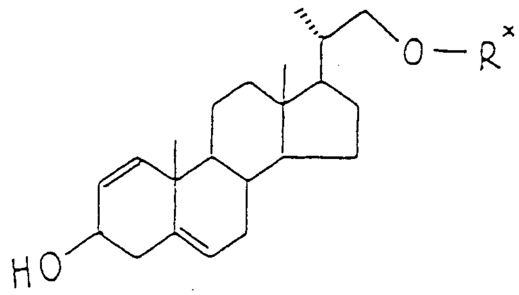

(Vb)

et dans ce dernier, le groupe 3-hydroxy est estérisé, en présence d'une base, avec un chlorure d'alkoxycarbonyle de la formule générale $R^{-}OC(O)Cl$ ($R^{-}$ est un résidu alkyle rectiligne ou à chaîne ramifiée avec 1 à 6 atomes d'azote) et ensuite l'ester obtenu est oxydé de manière analogue à l'étape de réaction VIIa respectivement VIIa'→VIIIa en position allyle en un composé correspondant de la formule générale VIIIb

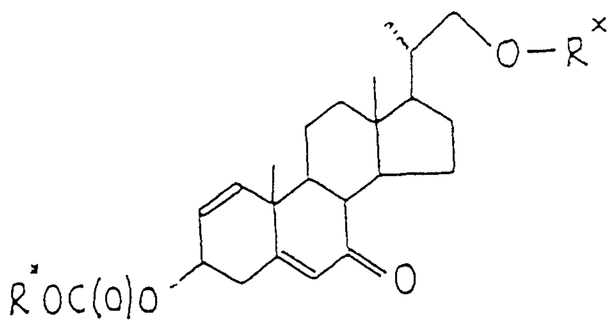

(VIIIb)

ce composé est époxydé de manière analogue à l'étape de réaction VIIIa→IXa en composé correspondant de la formule générale IXb

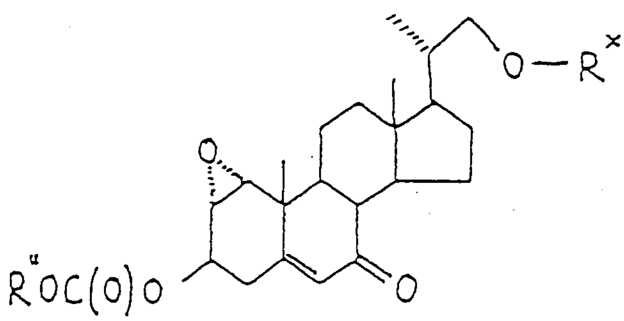

(IXb)

ce composé est transformé de manière analogue à l'étape de réaction IXa→Xa avec tosylhydrazon en tosylhydrazid correspondant de la formule générale Xb

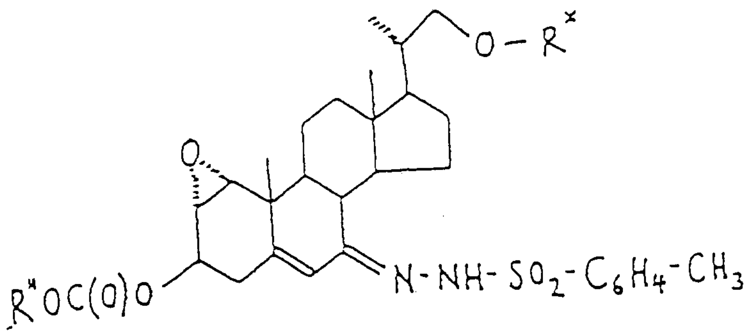

(Xb)

ceci est transformé ensuite de manière analogue à l'étape de réaction Xa→Ia avec de l'hydrure de lithium en un composé de la formule générale Ib

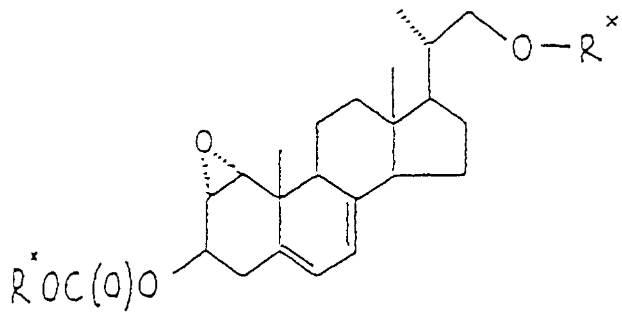

(Ib')

le groupe de protection $R^x$ est séparé dans des conditions douces, le composé formé 20-hydroxyméthyle de la formule générale Ib' est oxydé pour finir en 20-aldéhyde correspondant de la formule générale Ib

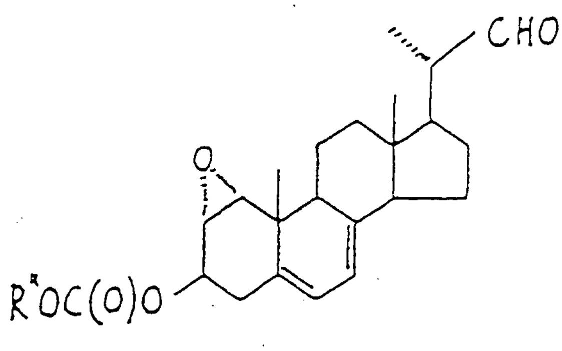

(Ib)

et si souhaité, l'ester 3-alkoxycarbonyle est transformé, par un procédé courant, en un autre substitut, appelé $R^1$ dans ce qui précède.

**3.** Produits intermédiaires de la formule générale VIIIa' et VIIIb

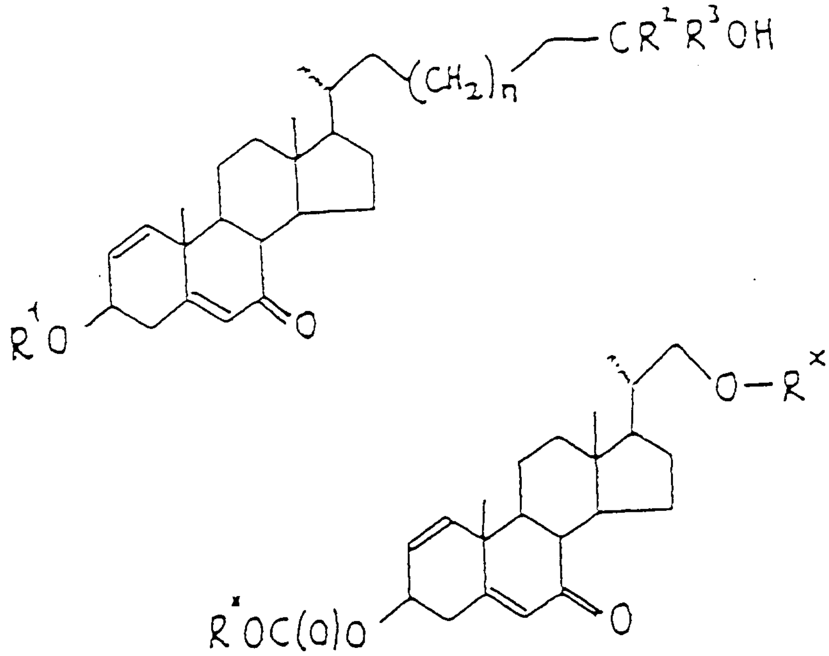

(VIII a')

(VIIIb)

dans laquelle
$R^1$, $R^2$, $R^3$ et n respectivement $R^-$ et $R^x$ présentent la signification indiquée précédemment dans la revendication 2, mais dans la formule générale VIIIa', si n = 1 et si $R^2$ et $R^3$ sont représentatifs d'un groupe méthyle, $R^1$ peut ne pas présenter d'oxygène ou d'adamantoyl.

**4.** Produits intermédiaires de la formule générale IXa et IXb

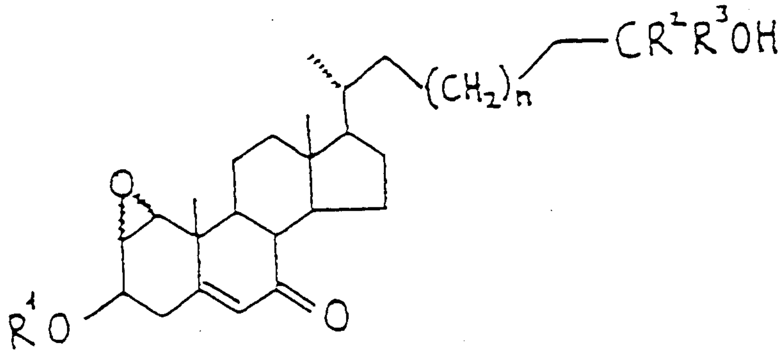

(IXa)

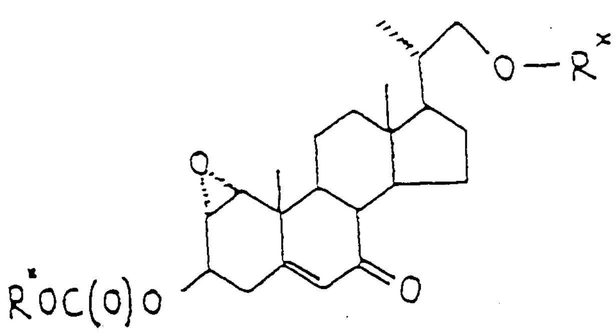

(IXb)

dans laquelle $R^1$, $R^2$, $R^3$ et n respectivement $R^-$ et $R^x$ présentent la signification indiquée précédemment dans la revendication 2.

**5.** Produits intermédiaires de la formule générale Xa et Xb

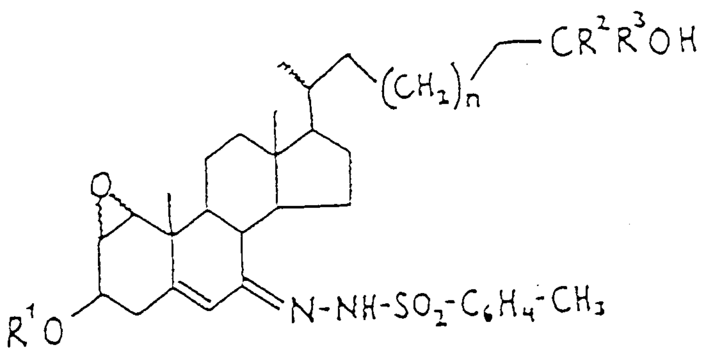

(Xa)

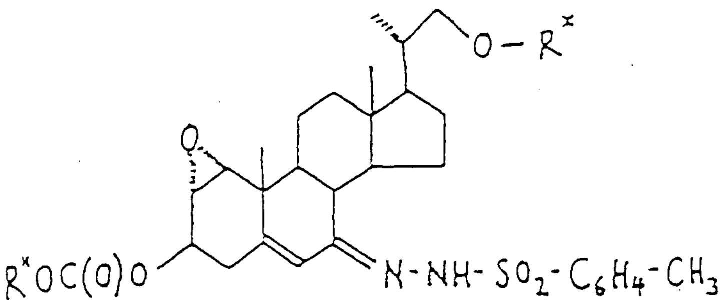

(Xb)

dans laquelle $R^1$, $R^2$, $R^3$ et $R^-$ et $R^x$ présentent là signification indiquée précédemment.